(19) Europäisches Patentamt
European Patent Office
Office européen des brevets

(11) EP 3 202 784 A1

(12) EUROPEAN PATENT APPLICATION

(43) Date of publication:
09.08.2017 Bulletin 2017/32

(51) Int Cl.:
C07K 14/725 (2006.01)    A61K 38/20 (2006.01)

(21) Application number: 16154713.8

(22) Date of filing: 08.02.2016

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(71) Applicant: Polybiocept AB
112 34 Stockholm (SE)

(72) Inventors:
• Mäurer, Markus
18444 Åkersberga (SE)
• Dodoo, Ernest
11234 Stockholm (SE)

(74) Representative: Patent- und Rechtsanwälte
Ullrich & Naumann
PartG mbB
Schneidmühlstrasse 21
69115 Heidelberg (DE)

(54) T-CELL RECEPTOR SEQUENCES FOR ACTIVE IMMUNOTHERAPY

(57) The present invention provides a protein, in particular a TCR comprising a Vβ amino acid sequence with a sequence identity of at 90 %, to a sequence selected from SEQ ID NO: 1 to 27, a T-cell comprising the TCR a method of selecting a T-cell product for use in active immunotherapy based on the identification of an Vβ amino acid sequence with a sequence identity of at 90 %, to a sequence selected from SEQ ID NO: 1 to 27 in a T-cell product.

Fig. 1

EP 3 202 784 A1

SEQUENCE LISTING

<110> PolyBioCept AB

<120> T-cell receptor sequences for active immunotherapy

<130> 7027/P/1005-EP

<160> 26

<170> PatentIn version 3.5

<210> 1
<211> 116
<212> PRT
<213> Homo sapiens

<400> 1

Gly Ala Val Val Ser Gln His Pro Ser Trp Val Ile Cys Lys Ser Gly
1               5                   10                  15


Thr Ser Val Lys Ile Glu Cys Arg Ser Leu Asp Phe Gln Ala Thr Thr
            20                  25                  30


Met Phe Trp Tyr Arg Gln Phe Pro Lys Gln Ser Leu Met Leu Met Ala
            35                  40                  45


Thr Ser Asn Glu Gly Ser Lys Ala Thr Tyr Glu Gln Gly Val Glu Lys
            50                  55                  60


Asp Lys Phe Leu Ile Asn His Ala Ser Leu Thr Leu Ser Thr Leu Thr
65                  70                  75                  80


Val Thr Ser Ala His Pro Glu Asp Ser Ser Phe Tyr Ile Cys Ser Ala
                85                  90                  95


Ala Thr Gly Asp Arg Pro Tyr Glu Gln Tyr Phe Gly Pro Gly Thr Arg
            100                 105                 110


Leu Thr Val Thr
            115


<210> 2
<211> 112
<212> PRT
<213> Homo sapiens

<400> 2

Asp Ala Gly Ile Thr Gln Ser Pro Arg His Lys Val Thr Glu Thr Gly
1               5                   10                  15


Thr Pro Val Thr Leu Arg Cys His Gln Thr Glu Asn His Arg Tyr Met

```
                20                      25                      30

    Tyr Trp Tyr Arg Gln Asp Pro Gly His Gly Leu Arg Leu Ile His Tyr
            35                  40                  45

    Ser Tyr Gly Val Lys Asp Thr Asp Lys Gly Glu Val Ser Asp Gly Tyr
            50                  55                  60

    Ser Val Ser Arg Ser Lys Thr Glu Asp Phe Leu Leu Thr Leu Glu Ser
    65                  70                  75                  80

    Ala Thr Ser Ser Gln Thr Ser Val Tyr Phe Cys Ala Ile Arg Thr Gly
                    85                  90                  95

    Ser Asp Asn Glu Gln Ser Phe Gly Pro Gly Thr Arg Leu Thr Val Leu
                100                 105                 110


<210>  3
<211>  113
<212>  PRT
<213>  Homo sapiens

<400>  3

    Glu Ala Glu Val Ala Gln Ser Pro Arg Tyr Lys Ile Thr Glu Lys Ser
    1               5                   10                  15

    Gln Ala Val Ala Phe Trp Cys Asp Pro Ile Ser Gly His Ala Thr Leu
                    20                  25                  30

    Tyr Trp Tyr Arg Gln Ile Leu Gly Gln Gly Pro Glu Leu Leu Val Gln
            35                  40                  45

    Phe Gln Asp Glu Ser Val Val Asp Asp Ser Gln Leu Pro Lys Asp Arg
            50                  55                  60

    Phe Ser Ala Glu Arg Leu Lys Gly Val Asp Ser Thr Leu Lys Ile Gln
    65                  70                  75                  80

    Pro Ala Glu Leu Gly Asp Ser Ala Met Tyr Leu Cys Ala Ser Arg Tyr
                    85                  90                  95

    Thr Gly Ser Ile Glu Gln Phe Phe Gly Pro Gly Thr Arg Leu Thr Val
                100                 105                 110

    Leu


<210>  4
```

```
<211>   115
<212>   PRT
<213>   Homo sapiens

<400>   4

Glu Ala Gln Val Thr Gln Asn Pro Arg Tyr Leu Ile Thr Val Thr Gly
1               5                   10                  15

Lys Lys Leu Thr Val Thr Cys Ser Gln Asn Met Asn His Glu Tyr Met
            20                  25                  30

Ser Trp Tyr Arg Gln Asp Pro Gly Leu Gly Leu Arg Gln Ile Tyr Tyr
            35                  40                  45

Ser Met Asn Val Glu Val Thr Asp Lys Gly Asp Val Pro Glu Gly Tyr
        50                  55                  60

Lys Val Ser Arg Lys Glu Lys Arg Asn Phe Pro Leu Ile Leu Glu Ser
65                  70                  75                  80

Pro Ser Pro Asn Gln Thr Ser Leu Tyr Phe Cys Ala Ser Ser Ala Gly
                85                  90                  95

Thr Ser Gly Val Thr Tyr Glu Gln Tyr Phe Gly Pro Gly Thr Arg Leu
            100                 105                 110

Thr Val Thr
            115


<210>   5
<211>   110
<212>   PRT
<213>   Homo sapiens

<400>   5

Asn Ala Gly Val Thr Gln Thr Pro Lys Phe Gln Val Leu Lys Thr Gly
1               5                   10                  15

Gln Ser Met Thr Leu Gln Cys Ala Gln Asp Met Asn His Asn Ser Met
            20                  25                  30

Tyr Trp Tyr Arg Gln Asp Pro Gly Met Gly Leu Arg Leu Ile Tyr Tyr
            35                  40                  45

Ser Ala Ser Glu Gly Thr Thr Asp Lys Gly Glu Val Pro Asn Gly Tyr
        50                  55                  60

Asn Val Ser Arg Leu Asn Lys Arg Glu Phe Ser Leu Arg Leu Glu Ser
65                  70                  75                  80
```

Ala Ala Pro Ser Gln Thr Ser Val Tyr Phe Cys Ala Ser Ser Thr Arg
                85                  90                  95

Phe Glu Gln Tyr Phe Gly Pro Gly Thr Arg Leu Thr Val Thr
                100                 105                 110


<210>   6
<211>   110
<212>   PRT
<213>   Homo sapiens

<400>   6

Asn Ala Gly Val Thr Gln Thr Pro Lys Phe Gln Val Leu Lys Thr Gly
1                   5                   10                  15

Gln Ser Met Thr Leu Gln Cys Ala Gln Asp Met Asn His Glu Tyr Met
                20                  25                  30

Ser Trp Tyr Arg Gln Asp Pro Gly Met Gly Leu Arg Leu Ile His Tyr
            35                  40                  45

Ser Val Gly Ala Gly Ile Thr Asp Gln Gly Glu Val Pro Asn Gly Tyr
        50                  55                  60

Asn Val Ser Arg Ser Thr Thr Glu Asp Phe Pro Leu Arg Leu Leu Ser
65                  70                  75                  80

Ala Ala Pro Ser Gln Thr Ser Val Tyr Phe Cys Ala Ser Ser Thr Arg
                85                  90                  95

Phe Glu Gln Tyr Phe Gly Pro Gly Thr Arg Leu Thr Val Thr
                100                 105                 110


<210>   7
<211>   110
<212>   PRT
<213>   Homo sapiens

<400>   7

Asn Ala Gly Val Thr Gln Thr Pro Lys Phe Arg Ile Leu Lys Ile Gly
1                   5                   10                  15

Gln Ser Met Thr Leu Gln Cys Thr Gln Asp Met Asn His Asn Tyr Met
                20                  25                  30

Tyr Trp Tyr Arg Gln Asp Pro Gly Met Gly Leu Lys Leu Ile Tyr Tyr
            35                  40                  45

```
Ser Val Gly Ala Gly Ile Thr Asp Lys Gly Glu Val Pro Asn Gly Tyr
    50                  55                  60

Asn Val Ser Arg Ser Thr Thr Glu Asp Phe Pro Leu Arg Leu Glu Leu
65                  70                  75                  80

Ala Ala Pro Ser Gln Thr Ser Val Tyr Phe Cys Ala Ser Ser Thr Arg
                85                  90                  95

Phe Glu Gln Tyr Phe Gly Pro Gly Thr Arg Leu Thr Val Thr
            100                 105                 110


<210>   8
<211>   116
<212>   PRT
<213>   Homo sapiens

<400>   8

Asp Ala Gly Val Ile Gln Ser Pro Arg His Glu Val Thr Glu Met Gly
1               5                   10                  15

Gln Glu Val Thr Leu Arg Cys Lys Pro Ile Ser Gly His Asn Ser Leu
            20                  25                  30

Phe Trp Tyr Arg Gln Thr Met Met Arg Gly Leu Glu Leu Leu Ile Tyr
        35                  40                  45

Phe Asn Asn Asn Val Pro Ile Asp Asp Ser Gly Met Pro Glu Asp Arg
    50                  55                  60

Phe Ser Ala Lys Met Pro Asn Ala Ser Phe Ser Thr Leu Lys Ile Gln
65                  70                  75                  80

Pro Ser Glu Pro Arg Asp Ser Ala Val Tyr Phe Cys Ala Ser Ser Ser
                85                  90                  95

Arg Arg Asp His Thr Tyr Asn Gly Gln Phe Phe Gly Pro Gly Thr Arg
                100                 105                 110

Leu Thr Val Leu
        115


<210>   9
<211>   115
<212>   PRT
<213>   Homo sapiens

<400>   9
```

```
Asp Ala Gly Val Ile Gln Ser Pro Arg His Glu Val Thr Glu Met Gly
1               5                   10                  15

Gln Glu Val Thr Leu Arg Cys Lys Pro Ile Ser Gly His Asp Tyr Leu
                20                  25                  30

Phe Trp Tyr Arg Gln Thr Met Met Arg Gly Leu Glu Leu Leu Ile Tyr
            35                  40                  45

Phe Asn Asn Asn Val Pro Ile Asp Asp Ser Gly Met Pro Glu Asp Arg
        50                  55                  60

Phe Ser Ala Lys Met Pro Asn Ala Ser Phe Ser Thr Leu Lys Ile Gln
65                  70                  75                  80

Pro Ser Glu Pro Arg Asp Ser Ala Val Tyr Phe Cys Ala Ser Ser Ser
                85                  90                  95

Arg Arg Asp His Thr Tyr Asn Gly Gln Phe Gly Pro Gly Thr Arg Leu
            100                 105                 110

Thr Val Leu
        115


<210>   10
<211>   113
<212>   PRT
<213>   Homo sapiens

<400>   10

Glu Ala Gln Val Thr Gln Asn Pro Arg Tyr Leu Ile Thr Val Thr Gly
1               5                   10                  15

Lys Lys Leu Thr Val Thr Cys Ser Gln Asn Met Asn His Glu Tyr Met
                20                  25                  30

Ser Trp Tyr Arg Gln Asp Pro Gly Leu Gly Leu Arg Gln Ile Tyr Tyr
            35                  40                  45

Ser Met Asn Val Glu Val Thr Asp Lys Gly Asp Val Pro Glu Gly Tyr
        50                  55                  60

Lys Val Ser Arg Lys Glu Lys Arg Asn Phe Pro Leu Ile Leu Glu Ser
65                  70                  75                  80

Pro Ser Pro Asn Gln Thr Ser Leu Tyr Phe Cys Ala Ser Ser Leu Gln
                85                  90                  95
```

Gly Ala Asn Tyr Gly Tyr Thr Phe Gly Ser Gly Thr Arg Leu Thr Val
              100                     105                 110

Val


<210> 11
<211> 121
<212> PRT
<213> Homo sapiens

<400> 11

Gly Ala Val Val Ser Gln His Pro Ser Trp Val Ile Cys Lys Ser Gly
1                   5                   10                  15

Thr Ser Val Lys Ile Glu Cys Arg Ser Leu Asp Phe Gln Ala Thr Thr
              20                  25                  30

Met Phe Trp Tyr Arg Gln Phe Pro Lys Gln Ser Leu Met Leu Met Ala
          35                  40                  45

Thr Ser Asn Glu Gly Ser Lys Ala Thr Tyr Glu Gln Gly Val Glu Lys
      50                  55                  60

Asp Lys Phe Leu Ile Asn His Ala Ser Leu Thr Leu Ser Thr Leu Thr
65                  70                  75                  80

Val Thr Ser Ala His Pro Glu Asp Ser Ser Phe Tyr Ile Cys Ser Ala
              85                  90                  95

Arg Val Ile Pro Ser Gly Gly Val Val Gln Gly Thr Asp Thr Gln Tyr
              100                 105                 110

Phe Gly Pro Gly Thr Arg Leu Thr Val
          115                 120


<210> 12
<211> 114
<212> PRT
<213> Homo sapiens

<400> 12

Lys Ala Gly Val Thr Gln Thr Pro Arg Tyr Leu Ile Lys Thr Arg Gly
1                   5                   10                  15

Gln Gln Val Thr Leu Ser Cys Ser Pro Ile Ser Gly His Arg Ser Val
              20                  25                  30

Ser Trp Tyr Gln Gln Thr Pro Gly Gln Gly Leu Gln Phe Leu Phe Glu

```
                    35                        40                        45

        Tyr Phe Ser Glu Thr Gln Arg Asn Lys Gly Asn Phe Pro Gly Arg Phe
            50                      55                      60

        Ser Gly Arg Gln Phe Ser Asn Ser Arg Ser Glu Met Asn Val Ser Thr
        65                      70                      75                      80

        Leu Glu Leu Gly Asp Ser Ala Leu Tyr Leu Cys Ala Ser Ser Leu Ser
                        85                      90                      95

        Trp Asp Lys Ser Tyr Glu Gln Tyr Phe Gly Pro Gly Thr Arg Leu Thr
                        100                     105                     110

        Val Thr


        <210>  13
        <211>  117
        <212>  PRT
        <213>  Homo sapiens

        <400>  13

        Glu Ala Glu Val Ala Gln Ser Pro Arg Tyr Lys Ile Thr Glu Lys Ser
        1               5                       10                      15

        Gln Ala Val Ala Phe Trp Cys Asp Pro Ile Ser Gly His Ala Thr Leu
                        20                      25                      30

        Tyr Trp Tyr Arg Gln Ile Leu Gly Gln Gly Pro Glu Leu Leu Val Gln
                35                      40                      45

        Phe Gln Asp Glu Ser Val Val Asp Asp Ser Gln Leu Pro Lys Asp Arg
                50                      55                      60

        Phe Ser Ala Glu Arg Leu Lys Gly Val Asp Ser Thr Leu Lys Ile Gln
        65                      70                      75                      80

        Pro Ala Glu Leu Gly Asp Ser Ala Met Tyr Leu Cys Ala Ser Ser Leu
                        85                      90                      95

        Ser Arg Leu Ala Leu Phe Ser Tyr Glu Gln Tyr Phe Gly Pro Gly Thr
                        100                     105                     110

        Arg Leu Thr Val Thr
                        115


        <210>  14
```

<211> 114
<212> PRT
<213> Homo sapiens

<400> 14

Asp Ser Gly Val Thr Gln Thr Pro Lys His Leu Ile Thr Ala Thr Gly
1               5                   10                  15

Gln Arg Val Thr Leu Arg Cys Ser Pro Arg Ser Gly Asp Leu Ser Val
                20                  25                  30

Tyr Trp Tyr Gln Gln Ser Leu Asp Gln Gly Leu Gln Phe Leu Ile Gln
            35                  40                  45

Tyr Tyr Asn Gly Glu Glu Arg Ala Lys Gly Asn Ile Leu Glu Arg Phe
        50                  55                  60

Ser Ala Gln Gln Phe Pro Asp Leu His Ser Glu Leu Asn Leu Ser Ser
65                  70                  75                  80

Leu Glu Leu Gly Asp Ser Ala Leu Tyr Phe Cys Ala Ser Ser Val Gln
                85                  90                  95

Gly Val Ser Gln Glu Thr Gln Tyr Phe Gly Pro Gly Thr Arg Leu Leu
                100                 105                 110

Val Leu


<210> 15
<211> 114
<212> PRT
<213> Homo sapiens

<400> 15

Asp Ala Gly Val Ile Gln Ser Pro Arg His Glu Val Thr Glu Met Gly
1               5                   10                  15

Gln Glu Val Thr Leu Arg Cys Lys Pro Ile Ser Gly His Asn Ser Leu
                20                  25                  30

Phe Trp Tyr Arg Gln Thr Met Met Arg Gly Leu Glu Leu Leu Ile Tyr
            35                  40                  45

Phe Asn Asn Asn Val Pro Ile Asp Asp Ser Gly Met Pro Glu Asp Arg
        50                  55                  60

Phe Ser Ala Lys Met Pro Asn Ala Ser Phe Ser Thr Leu Lys Ile Gln
65                  70                  75                  80

30

```
Pro Ser Glu Pro Arg Asp Ser Ala Val Tyr Phe Cys Ala Ser Ser Arg
                85                  90                  95

Arg Gly Ser Gly Asp Thr Gln Tyr Phe Gly Pro Gly Thr Arg Leu Thr
                100                 105                 110

Val Leu


<210>  16
<211>  114
<212>  PRT
<213>  Homo sapiens

<400>  16

Asp Ala Gly Val Ile Gln Ser Pro Arg His Glu Val Thr Glu Met Gly
1               5                   10                  15

Gln Glu Val Thr Leu Arg Cys Lys Pro Ile Ser Gly His Asp Tyr Leu
                20                  25                  30

Phe Trp Tyr Arg Gln Thr Met Met Arg Gly Leu Glu Leu Leu Ile Tyr
                35                  40                  45

Phe Asn Asn Asn Val Pro Ile Asp Asp Ser Gly Met Pro Glu Asp Arg
                50                  55                  60

Phe Ser Ala Lys Met Pro Asn Ala Ser Phe Ser Thr Leu Lys Ile Gln
65                  70                  75                  80

Pro Ser Glu Pro Arg Asp Ser Ala Val Tyr Phe Cys Ala Ser Ser Arg
                85                  90                  95

Arg Gly Ser Gly Asp Thr Gln Tyr Phe Gly Pro Gly Thr Arg Leu Thr
                100                 105                 110

Val Leu


<210>  17
<211>  112
<212>  PRT
<213>  Homo sapiens

<400>  17

Lys Ala Gly Val Thr Gln Thr Pro Arg Tyr Leu Ile Lys Thr Arg Gly
1               5                   10                  15
```

```
Gln Gln Val Thr Leu Ser Cys Ser Pro Ile Ser Gly His Arg Ser Val
            20                  25              30

Ser Trp Tyr Gln Gln Thr Pro Gly Gln Gly Leu Gln Phe Leu Phe Glu
            35              40                  45

Tyr Phe Ser Glu Thr Gln Arg Asn Lys Gly Asn Phe Pro Gly Arg Phe
        50                  55                  60

Ser Gly Arg Gln Phe Ser Asn Ser Arg Ser Glu Met Asn Val Ser Thr
65                  70                  75                  80

Leu Glu Leu Gly Asp Ser Ala Leu Tyr Leu Cys Ala Ser Ser Thr Asp
                85                  90                  95

Ser Asn Thr Glu Ala Phe Phe Gly Gln Gly Thr Arg Leu Thr Val Val
            100                 105                 110
```

```
<210>  18
<211>  114
<212>  PRT
<213>  Homo sapiens

<400>  18
```

```
Asp Val Lys Val Thr Gln Ser Ser Arg Tyr Leu Val Lys Arg Thr Gly
1               5                   10                  15

Glu Lys Val Phe Leu Glu Cys Val Gln Asp Met Asp His Glu Asn Met
            20                  25                  30

Phe Trp Tyr Arg Gln Asp Pro Gly Leu Gly Leu Arg Leu Ile Tyr Phe
            35              40                  45

Ser Tyr Asp Val Lys Met Lys Glu Lys Gly Asp Ile Pro Glu Gly Tyr
        50                  55                  60

Ser Val Ser Arg Glu Lys Lys Glu Arg Phe Ser Leu Ile Leu Glu Ser
65                  70                  75                  80

Ala Ser Thr Asn Gln Thr Ser Met Tyr Leu Cys Ala Ser Ser Phe Gln
                85                  90                  95

Thr Ala His Thr Asp Thr Gln Tyr Phe Gly Pro Gly Thr Arg Leu Thr
            100                 105                 110

Val Leu
```

```
<210>  19
<211>  112
<212>  PRT
<213>  Homo sapiens

<400>  19

Glu Ala Gly Val Thr Gln Phe Pro Ser His Ser Val Ile Glu Lys Gly
1               5                   10                  15


Gln Thr Val Thr Leu Arg Cys Asp Pro Ile Ser Gly His Asp Asn Leu
                20                  25                  30


Tyr Trp Tyr Arg Arg Val Met Gly Lys Glu Ile Lys Phe Leu Leu His
            35                  40                  45


Phe Val Lys Glu Ser Lys Gln Asp Glu Ser Gly Met Pro Asn Asn Arg
            50                  55                  60


Phe Leu Ala Glu Arg Thr Gly Gly Thr Tyr Ser Thr Leu Lys Val Gln
65                  70                  75                  80


Pro Ala Glu Leu Glu Asp Ser Gly Val Tyr Phe Cys Ala Ser Ser Leu
                85                  90                  95


Arg Asp Ser Asp Glu Ala Phe Phe Gly Gln Gly Thr Arg Leu Thr Val
            100                 105                 110


<210>  20
<211>  113
<212>  PRT
<213>  Homo sapiens

<400>  20

Asn Ala Gly Val Thr Gln Thr Pro Lys Phe Arg Val Leu Lys Thr Gly
1               5                   10                  15


Gln Ser Met Thr Leu Leu Cys Ala Gln Asp Met Asn His Glu Tyr Met
                20                  25                  30


Tyr Trp Tyr Arg Gln Asp Pro Gly Met Gly Leu Arg Leu Ile His Tyr
            35                  40                  45


Ser Val Gly Glu Gly Thr Thr Ala Lys Gly Glu Val Pro Asp Gly Tyr
            50                  55                  60


Asn Val Ser Arg Leu Lys Lys Gln Asn Phe Leu Leu Gly Leu Glu Ser
65                  70                  75                  80
```

33

Ala Ala Pro Ser Gln Thr Ser Val Tyr Phe Cys Ala Ser Ser Leu Gln
            85                     90                   95

Gly Arg Val Asp Glu Gln Phe Phe Gly Pro Gly Thr Arg Leu Thr Val
           100                105                110

Leu

<210> 21
<211> 116
<212> PRT
<213> Homo sapiens

<400> 21

Gly Ala Val Val Ser Gln His Pro Ser Trp Val Ile Cys Lys Ser Gly
1            5                10                15

Thr Ser Val Lys Ile Glu Cys Arg Ser Leu Asp Phe Gln Ala Thr Thr
           20                25                30

Met Phe Trp Tyr Arg Gln Phe Pro Lys Gln Ser Leu Met Leu Met Ala
        35                40                45

Thr Ser Asn Glu Gly Ser Lys Ala Thr Tyr Glu Gln Gly Val Glu Lys
        50                55                60

Asp Lys Phe Leu Ile Asn His Ala Ser Leu Thr Leu Ser Thr Leu Thr
65              70                75                80

Val Thr Ser Ala His Pro Glu Asp Ser Ser Phe Tyr Ile Cys Ser Ala
             85                90                95

Arg Asp Gly Thr Leu Asn Thr Glu Ala Phe Phe Gly Gln Gly Thr Arg
           100                105                110

Leu Thr Val Val
        115

<210> 22
<211> 118
<212> PRT
<213> Homo sapiens

<400> 22

Gly Ala Val Val Ser Gln His Pro Ser Trp Val Ile Cys Lys Ser Gly
1            5                10                15

Thr Ser Val Lys Ile Glu Cys Arg Ser Leu Asp Phe Gln Ala Thr Thr

                  20                    25                  30

```
Met Phe Trp Tyr Arg Gln Phe Pro Lys Gln Ser Leu Met Leu Met Ala
        35                  40                  45

Thr Ser Asn Glu Gly Ser Lys Ala Thr Tyr Glu Gln Gly Val Glu Lys
    50                  55                  60

Asp Lys Phe Leu Ile Asn His Ala Ser Leu Thr Leu Ser Thr Leu Thr
65                  70                  75                  80

Val Thr Ser Ala His Pro Glu Asp Ser Ser Phe Tyr Ile Cys Ser Ala
                85                  90                  95

Arg Pro Leu Arg Asp Arg Val Ala His Gly Tyr Thr Phe Gly Ser Gly
        100                 105                 110

Thr Arg Leu Thr Val Val
        115


<210>  23
<211>  115
<212>  PRT
<213>  Homo sapiens


<400>  23

Asp Gly Gly Ile Thr Gln Ser Pro Lys Tyr Leu Phe Arg Lys Glu Gly
1               5                   10                  15

Gln Asn Val Thr Leu Ser Cys Glu Gln Asn Leu Asn His Asp Ala Met
        20                  25                  30

Tyr Trp Tyr Arg Gln Asp Pro Gly Gln Gly Leu Arg Leu Ile Tyr Tyr
        35                  40                  45

Ser Gln Ile Val Asn Asp Phe Gln Lys Gly Asp Ile Ala Glu Gly Tyr
    50                  55                  60

Ser Val Ser Arg Glu Lys Lys Glu Ser Phe Pro Leu Thr Val Thr Ser
65                  70                  75                  80

Ala Gln Lys Asn Pro Thr Ala Phe Tyr Leu Cys Ala Ser Ser Ile Leu
                85                  90                  95

Ala Phe Arg Ala Gly Glu Thr Gln Tyr Phe Gly Pro Gly Thr Arg Leu
        100                 105                 110

Leu Val Leu
```

115

```
<210>  24
<211>  113
<212>  PRT
<213>  Homo sapiens

<400>  24

Gly Ala Gly Val Ser Gln Ser Pro Arg Tyr Lys Val Thr Lys Arg Gly
1               5                   10                  15


Gln Asp Val Ala Leu Arg Cys Asp Pro Ile Ser Gly His Val Ser Leu
            20                  25                  30


Tyr Trp Tyr Arg Gln Ala Leu Gly Gln Gly Pro Glu Phe Leu Thr Tyr
        35                  40                  45


Phe Asn Tyr Glu Ala Gln Gln Asp Lys Ser Gly Leu Pro Asn Asp Arg
    50                  55                  60


Phe Ser Ala Glu Arg Pro Glu Gly Ser Ile Ser Thr Leu Thr Ile Gln
65                  70                  75                  80


Arg Thr Glu Gln Arg Asp Ser Ala Met Tyr Arg Cys Ala Ser Ser Gln
                85                  90                  95


Gly Pro Asn Tyr Glu Gln Tyr Phe Gly Pro Gly Thr Arg Leu Thr Val
            100                 105                 110


Thr



<210>  25
<211>  115
<212>  PRT
<213>  Homo sapiens

<400>  25

Gly Ala Gly Val Ser Gln Ser Pro Ser Asn Lys Val Thr Glu Lys Gly
1               5                   10                  15


Lys Asp Val Glu Leu Arg Cys Asp Pro Ile Ser Gly His Thr Ala Leu
            20                  25                  30


Tyr Trp Tyr Arg Gln Ser Leu Gly Gln Gly Leu Glu Phe Leu Ile Tyr
        35                  40                  45


Phe Gln Gly Asn Ser Ala Pro Asp Lys Ser Gly Leu Pro Ser Asp Arg
    50                  55                  60
```

```
Phe Ser Ala Glu Arg Thr Gly Gly Ser Val Ser Thr Leu Thr Ile Gln
65              70              75                      80


Arg Thr Gln Gln Glu Asp Ser Ala Val Tyr Leu Cys Ala Ser Ser Phe
            85                  90                  95


Gly Leu Ala Gly Ala Asn Glu Gln Phe Phe Gly Pro Gly Thr Arg Leu
            100             105             110


Thr Val Leu
        115


<210>   26
<211>   116
<212>   PRT
<213>   Homo sapiens

<400>   26

Gly Ala Gly Val Ser Gln Ser Pro Ser Asn Lys Val Thr Glu Lys Gly
1               5               10                  15


Lys Asp Val Glu Leu Arg Cys Asp Pro Ile Ser Gly His Thr Ala Leu
            20                  25                  30


Tyr Trp Tyr Arg Gln Ser Leu Gly Gln Gly Leu Glu Phe Leu Ile Tyr
        35                  40                  45


Phe Gln Gly Asn Ser Ala Pro Asp Lys Ser Gly Leu Pro Ser Asp Arg
        50                  55                  60


Phe Ser Ala Glu Arg Thr Gly Gly Ser Val Ser Thr Leu Thr Ile Gln
65              70              75                      80


Arg Thr Gln Gln Glu Asp Ser Ala Val Tyr Leu Cys Ala Ser Ser Ser
            85                  90                  95


Arg Leu Ala Gly Thr Tyr Asn Glu Gln Phe Phe Gly Pro Gly Thr Arg
            100             105             110


Leu Thr Val Leu
        115
```

**Description**

**FIELD OF THE INVENTION**

The present invention relates to proteins comprising a Vbeta sequence with an amino acid sequence with a sequence identity of at 90 %, to a sequence for use in immunotherapy.

**BACKGROUND OF THE INVENTION**

One of the most promising advances for the treatment of cancer is a new therapeutic class called active cellular immunotherapy (ACI). Active immunotherapies stimulate the patient's immune system with the intent of promoting an antigen specific anti-tumor effect using the body's own immune cells. In addition active immunotherapies seek to create durable anti-tumor response that can protect against minimal residual disease and tumor recurrences as well as against potentially pre-malignant tumor lesions.

Clinically relevant and long-term remissions have been achieved in patients with melanoma using T-cells directed against tumors (tumor reactive T-cells) (1, 2). These approaches usually rely on the harvesting of tumor infiltrating lymphocytes (TIL) from tumor lesions or T-cells from peripheral blood.

Clinical (antitumor) efficacy appears to be mediated by CD8+, or by CD4+ T-cells residing preferentially in central memory cells, defined by CD45RA-CCR7+, defined ex vivo from patients responding to T-cell based therapy. The phenotype of such T-cells is determined by the ex vivo expanded T-cell population, as well as by host factors after adoptive transfer. A diverse population of T-cells targeting cancer cells may be advantageous for effective immune responses, including long-term memory T-cells, yet also T-cells that can immediately react to (cancer) target cells and produce anti-tumor directed immune responses, including terminally differentiated T-cells that express cytolytic molecules, such as granzyme and perforin (3, 4). Long term-memory immune memory is in part determined by the increased proliferation potential and half-life that can be measured by the telomere length (5, 6).

In WO 2015/189357 A1 the inventors have described a novel cytokine cocktail containing IL-2, IL-15 and IL-21 for the expansion of lymphocytes, in particular T-cells. T-cell populations obtained by expansion in the presence of the cytokine cocktail are able to not only recognize autologous tumor calls but also kill such tumor cells in vitro.

WO 2015/189357 A1 describes a variety of T-cell products obtained from an expansion of T-cells from the tumor, i.e. tumor infiltrating lymphocytes (TILs) or peripheral blood of patients with pancreatic cancer or glioblastoma. With the expansion protocol using the cytokine cocktail containing IL-2, IL-15 and IL-21 it is possible to produce several T-cell products in parallel. These T-cell products in general show a phenotype distribution advantageous for the active immune therapy.

However, besides the phenotype distribution and the in vitro testing of the T-cell product the clinician at present does not have further determinants for the selection for a T-cell product for insertion into the patient.

**SUMMARY OF THE INVENTION**

The present invention is *inter alia* based on the identification TCR sequences that are responsible for recognition and killing of diseased tissue cells. TCRs with the respective TCR sequences target tumor associated antigens and T-cells comprising the TCRs are therefore valuable therapeutic tools and a detection of the TCR sequences can be used for evaluation of T-cell products, i.e. an in vitro expanded T-cell population for therapeutic use.

Thus, according to a first aspect the invention provides a protein comprising a Vβ amino acid sequence with a sequence identity of at 90 % to a sequence selected from SEQ ID NO: 1 to 27.

According to a second aspect the invention provides a T-cell comprising a TCR which is a protein according to the first aspect. T-cell according to the second aspect may in particular be used in the treatment of cancer.

Furthermore according to a third aspect the invention provides a method of selecting a T-cell product for use in active immunotherapy in a patient, wherein the method comprises the following steps:

a) providing one or more T-cell products;
b) identifying the presence of a TCR comprising a sequence with a homology of at least 95 % to the sequence selected from SEQ ID NO: 1 to 27 in the one or more T-cell products; and
c) selecting the T-cell product comprising a TCR identified in step b) for use in therapy.

**DESCRIPTION OF THE DRAWINGS**

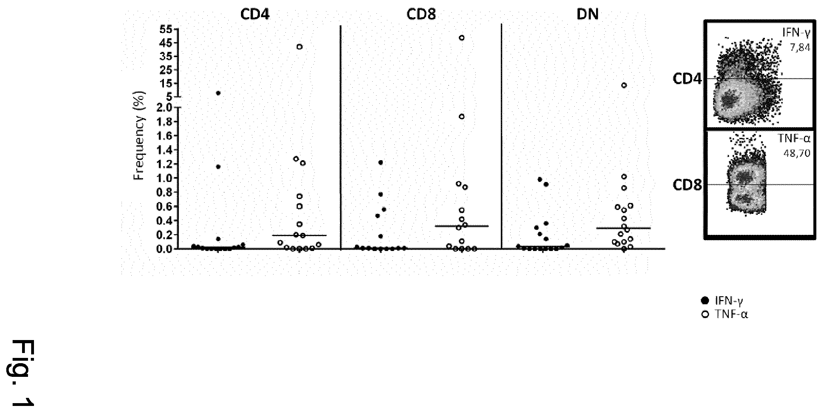

Figure 1    shows the frequency of IFNγ and TNFα producing TIL in response to autologous tumor cells. Numbers represent the percentage of T-cells specifically producing IFNγ (black circles) and TNFα (open circles) in the parental CD8+, CD4+, or (CD3+), CD4-CD8- TIL subsets. Each circle represent TIL from an individual patient. Medium values have been subtracted. Right panel: example flow cytometric analysis of CD4+ and CD8+ TIL producing cytokines directed against autologous tumor cells.

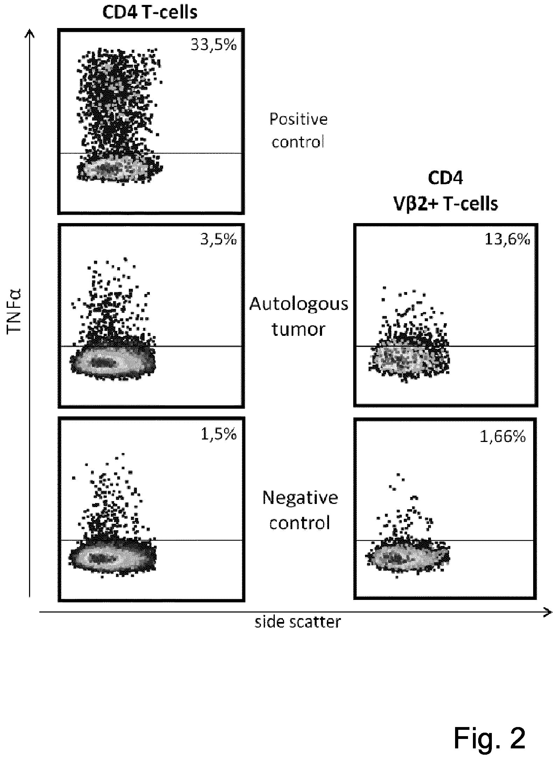

Figure 2    shows the link of expanded Vβ families in TIL with the recognition of autologous tumor cells, in particular the frequency of TNFalpha producing T-cells in CD3+CD4+ T-cells. Top left: positive control. Middle panel. Exposure to autologous tumor cells. TIL gated on CD3+CD4+ T-cells: 3.5% cells produce TNFalpha; right: 13.6% TNFα producing TIL in DNCD3+CD4+ Vβ2+ T- cells from patient GBM-D. Bottom panel. Left: negative control in CD3+CD4+ TIL. Right: CD3+CD4+Vβ2+ TIL.

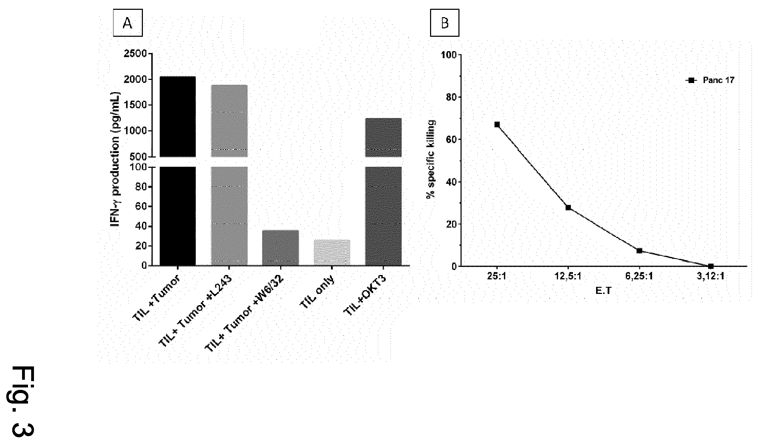

Figure 3    shows (A) the IFNγ production in TILs after co-culture with autologous tumor cells (Panc 9). CD8+ TILs (Vβ13.2 dominant) recognize the autologous tumor and IFNγ production can be inhibited by the anti-MHC-I antibody (W6/32), yet no with the anti-HLA-DR directed mAb. (B) The Recognition of the autologous tumor cell line in Panc 17 TIL using a standard Chromium 51 release assay.

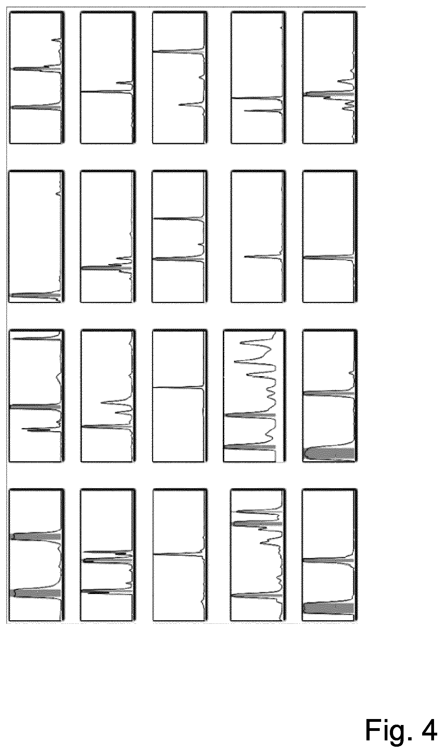

Figure 4    shows the results of a TCR CDR3 analysis of frequent TCR Vβ families after a 4 week expansion using IL-2, IL-15 and IL-21.

## DETAILED DESCRIPTION OF THE INVENTION

"Disease associated antigens" according to the invention are antigens involved in a disease. Accordingly, clinically relevant antigens can be tumor-associated antigens TAA.

According to the invention an "antigen" (Ag) is any structural substance which serves as a target for the receptors of an adaptive immune response, TCR or antibody, respectively. Antigens are in particular proteins, polysaccharides, lipids and substructures thereof such as peptides. Lipids and nucleic acids are in particular antigenic when combined with proteins or polysaccharides.

"Tumor associated antigens" or "TAA" according to the invention are antigens that are presented by MHC I or MHC II molecules or non-classical MHC molecules on the surface of tumor cells. As used herein TAA includes "tumor-specific antigens" which are found only on the surface of tumor cells, but not on the surface of normal cells.

As used herein, "interleukin 2" or "IL-2" refers to human IL-2 as defined by SEQ ID NO: 1 and functional equivalents thereof. Functional equivalents of IL-2 include relevant substructures or fusion proteins of IL-2 that remain the functions of IL-2. Accordingly, the definition IL-2 comprises any protein with a sequence identity to SEQ ID NO: 1 of at least 80 %, preferably at least 90 %, more preferably at least 95 %, most preferably at least 98 %. Recombinant human IL-2 produced in E. coli as a single, non-glycosylated polypeptide chain with 134 amino acids and having a molecular mass of 15 kDa is commercially available in lyophilized form from Prospec as CYT-209.

As used herein, "interleukin 15" or "IL-15" refer to human IL-15 and functional equivalents thereof. Functional equivalents of IL-15 include relevant substructures or fusion proteins of IL-15 that remain the functions of IL-15. Accordingly the definition IL-15 comprises any protein with a sequence identity to SEQ ID NO: 2 of at least 80 %, preferably at least 90 %, more preferably at least 95 %, most preferably at least 98 %. Recombinant human IL-15 produced in E. coli as a single, non-glycosylated polypeptide chain with 114 amino acids (and an N-terminal Methionine) and having a molecular mass of 12.8 kDa is commercially available in lyophilized form from Prospec as CYT-230.

As used herein, "interleukin 21" or "IL-21" refer to human IL-21 and functional equivalents thereof. Functional equivalents of IL-21 included relevant substructures or fusion proteins of IL-21 that remain the functions of IL-21. Accordingly the definition IL-21 comprises any protein with a sequence identity to SEQ ID NO: 3 of at least 80 %, preferably at least 90 %, more preferably at least 95 %, most preferably at least 98 %. Recombinant human IL-21 produced in E. coli as a single, non-glycosylated polypeptide chain with 132 amino acids and having a molecular mass of 15 kDa is commercially available in lyophilized form from Prospec as CYT-408.

A "peptide" as used herein may be composed of any number of amino acids of any type, preferably naturally occurring amino acids, which, preferably, are linked by peptide bonds. In particular, a peptide comprises at least 3 amino acids, preferably at least 5, at least 7, at least 9, at least 12, or at least 15 amino acids. Furthermore, there is no upper limit for the length of a peptide. However, preferably, a peptide according to the invention does not exceed a length of 500 amino acids, more preferably it does not exceed a length of 300 amino acids; even more preferably it is not longer than 250 amino acids.

Thus, the term "peptide" includes "oligopeptides", which usually refer to peptides with a length of 2 to 10 amino acids, and "polypeptides" which usually refer to peptides with a length of more than 10 amino acids.

The term "protein" refers to a peptide with at least 60, at least 80, preferably at least 100 amino acids.

The term "fusion protein" according to the invention relates to proteins created through the joining of two or more genes, cDNAs or sequences that originally coded for separate proteins/peptides. The genes may be naturally occurring in the same organism or different organisms or may synthetic polynucleotides.

The relatedness between two amino acid sequences or between two nucleotide sequences is described by the parameter "sequence identity". For purposes of the present invention, the degree of sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et a/., 2000, Trends Genet. 16: 276-277), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using thenobrief option) is used as the percent identity and is calculated as follows:

$$\text{(Identical Residues x 100)/(Length of Alignment - Total Number of Gaps in Alignment)}$$

The transitional term "comprising", which is synonymous with "including," "containing," or "characterized by," is inclusive or open-ended and does not exclude additional, unrecited elements or method steps. The transitional phrase "consisting of" excludes any element, step, or ingredient not specified in the claim, except for impurities ordinarily associated therewith. When the phrase "consists of" appears in a clause of the body of a claim, rather than immediately following the preamble, it limits only the element set forth in that clause; other elements are not excluded from the claim as a whole. The transitional phrase "consisting essentially of" limits the scope of a claim to the specified materials or steps "and those that do not materially affect the basic and novel characteristic(s)" of the claimed invention. "A 'consisting essentially of' claim occupies a middle ground between closed claims that are written in a 'consisting of' format and fully open claims that are drafted in a 'comprising' format."

"Expansion" or "clonal expansion" as used herein means production of daughter cells all arising originally from a single cell. In a clonal expansion of lymphocytes, all progeny share the same antigen specificity.

A "T-cell product" as used herein refers to a population of T-cell for use in immune therapy.

"TIL" according to the invention refers to tumor infiltrating lymphocyte. These are lymphocytes, in particular T-cells predominantly found in a tumor.

"Clinical/biological relevance" as used herein relates to the ability of a T-cell to provide at least one of the following: containment of tumor cells, destruction of tumor cells, prevention of metastasis, stop of proliferation, stop of cellular activity, stop of progress of cells to malignant transformation, prevention of metastases and / or tumor relapse, including reprogramming of malignant cells to their non-malignant state; prevention and / or stop of negative clinical factors associated with cancer, such as malnourishment or immune suppression, stop of accumulation of mutations leading to immune escape and disease progression, including epigenetic changes, induction of long-term immune memory preventing spread of the disease or future malignant transformation affecting the target (potential tumor cells), including connective tissue and non-transformed cells that would favor tumor disease.

According to a first aspect the invention provides a protein comprising a Vβ amino acid sequence with a sequence identity of at 90 %, to a sequence selected from SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, and SEQ ID NO: 26. The sequences SEQ ID NO: 1 to 26 were identified in T-cell products obtained by expansion of T-cells and shown to be responsible for recognition and killing of autologous tumor cells. The sequences are shown in Tables 1 and 2.

Table 1: Vβ sequences found in TIL expanded from Glioblastoma

| GBM A-CD4 (SEQ ID NO: 1) |
| --- |
| GAVVSQHPSWVICKSGTSVKIECRSLDFQATTMFWYRQFPKQSLMLMATSNEGS KATYEQGVEKDKFLINHASLTLSTLTVTSAHPEDSSFYICSAATGDRPYEQYFGPG TRLTVT |
| **GBM A-CD8 A (SEQ ID NO: 2)** |

(continued)

| DAGITQSPRHKVTETGTPVTLRCHQTENHRYMYWYRQDPGHGLRLIHYSYGVKD TDKGEVSDGYSVSRSKTEDFLLTLESATSSQTSVYFCAIRTGSDNEQSFGPGTRL TVL |
|---|
| **GBM A-CD8 B (SEQ ID NO: 3)**<br><br>EAEVAQSPRYKITEKSQAVAFWCDPISGHATLYWYRQILGQGPELLVQFQDESVV DDSQLPKDRFSAERLKGVDSTLKIQPAELGDSAMYLCASRYTGSIEQFFGPGTRL TVL |
| **GBM F-CD4 (SEQ ID NO: 4)**<br><br>EAQVTQNPRYLITVTGKKLTVTCSQNMNHEYMSWYRQDPGLGLRQIYYSMNVEV TDKGDVPEGYKVSRKEKRNFPLILESPSPNQTSLYFCASSAGTSGVTYEQYFGPG TRLTVT |
| **GBM G-CD4 A (SEQ ID NO: 5)**<br><br>NAGVTQTPKFQVLKTGQSMTLQCAQDMNHNSMYWYRQDPGMGLRLIYYSASE GTTDKGEVPNGYNVSRLNKREFSLRLESAAPSQTSVYFCASSTRFEQYFGPGTR LTVT |
| **GBM G-CD4 B (SEQ ID NO: 6)**<br><br>NAGVTQTPKFQVLKTGQSMTLQCAQDMNHEYMSWYRQDPGMGLRLIHYSVGA GITDQGEVPNGYNVSRSTTEDFPLRLLSAAPSQTSVYFCASSTRFEQYFGPGTRL TVT |
| **GBM G CD4 C (SEQ ID NO: 7)**<br><br>NAGVTQTPKFRILKIGQSMTLQCTQDMNHNYMYWYRQDPGMGLKLIYYSVGAGI TDKGEVPNGYNVSRSTTEDFPLRLELAAPSQTSVYFCASSTRFEQYFGPGTRLTV T |
| **GBM H-CD8 A (SEQ ID NO: 8)**<br><br>DAGVIQSPRHEVTEMGQEVTLRCKPISGHNSLFWYRQTMMRGLELLIYFNNNVPI DDSGMPEDRFSAKMPNASFSTLKIQPSEPRDSAVYFCASSSRRDHTYNGQFFGP GTRLTVL |
| **GBM H-CD8 B (SEQ ID NO: 9)**<br><br>DAGVIQSPRHEVTEMGQEVTLRCKPISGHDYLFWYRQTMMRGLELLIYFNNNVPI DDSGMPEDRFSAKMPNASFSTLKIQPSEPRDSAVYFCASSSRRDHTYNGQFGP GTRLTVL |
| **GBM H-CD8 C (SEQ ID NO: 10)**<br><br>EAQVTQNPRYLITVTGKKLTVTCSQNMNHEYMSWYRQDPGLGLRQIYYSMNVEV TDKGDVPEGYKVSRKEKRNFPLILESPSPNQTSLYFCASSLQGANYGYTFGSGTR LTVV |
| **GBM I-CD4 (SEQ ID NO: 11)** |

(continued)

| |
|---|
| GAVVSQHPSWVICKSGTSVKIECRSLDFQATTMFWYRQFPKQSLMLMATSNEGS KATYEQGVEKDKFLINHASLTLSTLTVTSAHPEDSSFYICSARVIPSGGVVQGTDT QYFGPGTRLTVL |
| **GBM I-CD8 (SEQ ID NO: 12)** |
| KAGVTQTPRYLIKTRGQQVTLSCSPISGHRSVSWYQQTPGQGLQFLFEYFSETQ RNKGNFPGRFSGRQFSNSRSEMNVSTLELGDSALYLCASSLSWDKSYEQYFGP GTRLTVT |
| **GBM J-CD4 (SEQ ID NO: 13)** |
| EAEVAQSPRYKITEKSQAVAFWCDPISGHATLYWYRQILGQGPELLVQFQDESVV DDSQLPKDRFSAERLKGVDSTLKIQPAELGDSAMYLCASSLSRLALFSYEQYFGP GTRLTVT |

Table 2: Vβ sequences found in TIL expanded from Pancreas Cancer

| |
|---|
| **Panc1 - CD8 (SEQ ID NO: 14)** |
| DSGVTQTPKHLITATGQRVTLRCSPRSGDLSVYWYQQSLDQGLQFLIQYYNGEER AKGNILERFSAQQFPDLHSELNLSSLELGDSALYFCASSVQGVSQETQYFGPGTRL LVL |
| **Panc5 - CD4 A (SEQ ID NO: 15)** |
| DAGVIQSPRHEVTEMGQEVTLRCKPISGHNSLFWYRQTMMRGLELLIYFNNNVPID DSGMPEDRFSAKMPNASFSTLKIQPSEPRDSAVYFCASSRRGSGDTQYFGPGTR LTVL |
| **Panc5 - CD4 B (SEQ ID NO:16)** |
| DAGVIQSPRHEVTEMGQEVTLRCKPISGHDYLFWYRQTMMRGLELLIYFNNNVPID DSGMPEDRFSAKMPNASFSTLKIQPSEPRDSAVYFCASSRRGSGDTQYFGPGTR LTVL |
| **Panc7 - CD4 B (SEQ ID NO: 17)** |
| KAGVTQTPRYLIKTRGQQVTLSCSPISGHRSVSWYQQTPGQGLQFLFEYFSETQR NKGNFPGRFSGRQFSNSRSEMNVSTLELGDSALYLCASSTDSNTEAFFGQGTRLT VV |
| **Panc7 - CD8 B (SEQ ID NO: 18)** |
| DVKVTQSSRYLVKRTGEKVFLECVQDMDHENMFWYRQDPGLGLRLIYFSYDVKM KEKGDIPEGYSVSREKKERFSLILESASTNQTSMYLCASSFQTAHTDTQYFGPGTR LTVL |
| **Panc 8 - CD8 (SEQ ID NO: 19)** |
| EAGVTQFPSHSVIEKGQTVTLRCDPISGHDNLYWYRRVMGKEIKFLLHFVKESKQD ESGMPNNRFLAERTGGTYSTLKVQPAELEDSGVYFCASSLRDSDEAFFGQGTRLT V |
| **Panc 9 - CD8 (SEQ ID NO:20)** |

(continued)

| |
|---|
| NAGVTQTPKFRVLKTGQSMTLLCAQDMNHEYMYWYRQDPGMGLRLIHYSVGEG TTAKGEVPDGYNVSRLKKQNFLLGLESAAPSQTSVYFCASSLQGRVDEQFFGPGT RLTVL |
| **Panc 16 - CD4 A (SEQ ID NO: 21)** |
| GAVVSQHPSWVICKSGTSVKIECRSLDFQATTMFWYRQFPKQSLMLMATSNEGS KATYEQGVEKDKFLINHASLTLSTLTVTSAHPEDSSFYICSARDGTLNTEAFFGQGT RLTVV |
| **Panc 16 - CD4 B (SEQ ID NO: 22)** |
| GAVVSQHPSWVICKSGTSVKIECRSLDFQATTMFWYRQFPKQSLMLMATSNEGS KATYEQGVEKDKFLINHASLTLSTLTVTSAHPEDSSFYICSARPLRDRVAHGYTFGS GTRLTVV |
| **Panc 16 - CD8 A (SEQ ID NO:23)** |
| DGGITQSPKYLFRKEGQNVTLSCEQNLNHDAMYWYRQDPGQGLRLIYYSQIVNDF QKGDIAEGYSVSREKKESFPLTVTSAQKNPTAFYLCASSILAFRAGETQYFGPGTR LLVL |
| **Panc 16 - CD8 B (SEQ ID NO:24)** |
| GAGVSQSPRYKVTKRGQDVALRCDPISGHVSLYWYRQALGQGPEFLTYFNYEAQ QDKSGLPNDRFSAERPEGSISTLTIQRTEQRDSAMYRCASSQGPNYEQYFGPGTR LTVT |
| **Panc 17 - CD8 A (SEQ ID NO: 25)** |
| GAGVSQSPSNKVTEKGKDVELRCDPISGHTALYWYRQSLGQGLEFLIYFQGNSAP DKSGLPSDRFSAERTGGSVSTLTIQRTQQEDSAVYLCASSFGLAGANEQFFGPGT RLTVL |
| **Panc 17 - CD8 B (SEQ ID NO:26)** |
| GAGVSQSPSNKVTEKGKDVELRCDPISGHTALYWYRQSLGQGLEFLIYFQGNSAP DKSGLPSDRFSAERTGGSVSTLTIQRTQQEDSAVYLCASSSRLAGTYNEQFFGPG TRLTVL |

According to a preferred embodiment the amino acid sequence of the protein according to the invention has sequence identity is at least 95 % to any one of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, and SEQ ID NO: 26. More preferably the sequence identity is at least 98 % According to a preferred embodiment the sequence identity to any one of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, and SEQ ID NO: 26 is 100 %.

The protein may be a T-cell receptor (TCR) in a T-cell obtained by expansion of autologous T-cells. Alternatively the protein may be a recombinant protein. The methods for producing, i.e. constructing and expressing recombinant proteins are well known in the art (see e.g. (7) and (8). The recombinant protein may for example designed for introduction into a cell. The cell may be a T-cell., an NK-cell, NK-T-cell or any innate immune cell. Innate immune cells include innate lymphoid cells, MAIT cells or immune memory of effector cells suitable for long-term and effective anti-tumor reactivity. The recombinant protein may be a transgenic TCR, such as the "anti.NY-ESO-1 TCR" (see e.g. (9)).. Such a transgenic TCR may be virally or non-virally transferred into recipient cells. Protocols for the viral and non-viral transfer are known in the art. Examples for the transfer are described in (10), (11) and (12).

According to a second aspect the invention provides a T-cell comprising a TCR of to the first aspect. The T-cell comprising the TCR according to the first aspect is in particular suitable for treatment of cancer. The cancer preferably selected from acute lymphocytic cancer, acute myeloid leukemia, alveolar rhabdomyosarcoma, bone cancer, brain cancer, breast cancer, cancer of the anus, anal canal, or anorectum, cancer of the eye, cancer of the intrahepatic bile duct, cancer of the joints, cancer of the neck, gallbladder, or pleura, cancer of the nose, nasal cavity, or middle ear, cancer of the vulva, chronic lymphocytic leukemia, chronic myeloid cancer, cervical cancer, glioma, Hodgkin lymphoma, hypopharynx cancer, kidney cancer, larynx cancer, liver cancer, lung cancer, malignant mesothelioma, melanoma, multiple myeloma, nasopharynx cancer, non-Hodgkin lymphoma, ovarian cancer, peritoneum, omentum, mesentery cancer, pancreatic cancer, pharynx cancer, prostate cancer, rectal cancer, renal cancer, skin cancer, soft tissue cancer, testicular cancer, thyroid cancer, ureter cancer, urinary bladder cancer, and digestive tract cancer such as, e.g., esophageal cancer, gastric cancer, pancreatic cancer, stomach cancer, small intestine cancer, gastrointestinal carcinoid tumor, cancer of the oral cavity, colon cancer, and hepatobiliary cancer. The T-cell may preferably be used in the treatment of glioblastoma or pancreatic cancer.

As shown in the examples T-cell products comprising a TCR with a sequence selected from any of SEQ ID NO: 1 to 27 are able to recognize and kill tumor cells in vitro. Therefore, identification of the TCR in a T-cell product drastically improves the chance that the T-cell product is able to fight the cancer in vivo, in particular that the T-cell product is clinically/biologically relevant.

Thus, according to a third aspect the invention provides a method of selecting a T-cell product for use in active immunotherapy in a patient, wherein the method comprises the following steps:

    a) providing at one or more T-cell products;
    b) identifying the presence of a according to the first aspect in the one or more T-cell products; and
    c) selecting the T-cell product comprising a TCR identified in step b) for use in therapy.

The TCR According to one embodiment the method further comprises determining the clonality of the $V\beta$-family to which the TCR belongs and selecting the T-cell product in which the TCR is present and the $V\beta$ family to which the TCR belongs is monoclonal. Monoclonality of the TCR indicated a directed and specific expansion of the TCR.

According to one embodiment the method further comprises determining the percentage of the TCR and/or the $V\beta$ family to which the TCR belongs in the T-cell product and selecting the T-cell product in which the percentage of the TCR is the highest. In order provide clinical/biological relevance to the T-cell product the TCR should be present in a sufficient concentration.

The method according to third aspect may be used in particular in the treatment of cancer. Thus the method is preferably carried out with a patient suffering from cancer. The cancer may be for example one of the cancers listed above. Preferably the cancer is selected from glioblastoma and pancreas cancer.

According to one embodiment of the third aspect providing the one or more T-cell products is based on an expansion of T-cells according to the invention. More preferably the method comprises

    -    providing a body sample containing T-cells from the patient;
    -    culturing cells of the sample *in-vitro* in the presence of at least one cytokine,

The body sample can be taken from any part of the body that contains lymphocytes. Examples of body samples are peripheral blood, cord blood, bone marrow, lymph nodes, liver pleural effusion, thorax, abdominal cavity, synvial fluid, peritoneum, retroperitoneal space, thymus, and tumor. A lymphocyte sample derived from tumor is also referred as tumor infiltrating lymphocytes (TIL). "TIL" as used herein is short for "tumor infiltrating lymphocytes". TIL is any kind of lymphocyte that is located in, on or around a tumor.

Preferably the culturing for expansion of the T-cells occurs in the presence of IL-2, IL-15 and IL-21. According to a further embodiment, the concentration of IL-2 in the liquid composition is in the range of from 10 to 6000 U/ml. The International Unit (U) is the standard measure for an amount or IL-2. It is determined by its ability to induce the proliferation of CTLL-2 cells. The concentration of IL-2 is preferably in the range from 500 to 2000 U/ml. More preferably the concentration of IL-2 is in the range from 800 to 1100 U/ml. According to one embodiment the concentration of IL-15 is in the range of 0.1 to 100 ng/ml. Preferably, the concentration of IL-15 is in the range from 2 to 50 ng/ml, more preferably in the range from 5 to 20 ng/ml. The most preferred concentration is about 10 ng/ml. In a further embodiment the concentration of IL-21 is in the range from 0.1 ng/ml, preferably in the range from 2 to 50 ng/ml, more preferably in the range from 5 to 20 ng/ml.

According to one embodiment of the invention the culture medium of the first expansion step comprises at least one expansion antigen. The expansion antigen is a known disease associated antigen or a fragment, a mutant or a variant thereof. As used herein a "mutant" is defined as an amino acid sequence that differs from the reference sequence by an insertion, deletion or replacement of at least one amino acid. The expansion antigen is a TAA.

According to a preferred embodiment of the method according to the second aspect, the body sample is tumor and no expansion antigen is used in culturing.

The invention is further defined by the following examples.

## EXAMPLES

### Example 1 - Expansion of TIL and tumor cell culture from glioma patients

#### 1.1 Diagnosis and Patients:

16 patients (Supplementary Table 1) with gliomas were enrolled. The study was approved by the regional ethical review board at Karolinska Institutet, Stockholm, Sweden (Dnr: 2013/576-31).

#### 1.2 TIL expansion:

Glioma tumor tissue was harvested in the course of tumor surgery and dissected into fragments (approximately 1-2mm$^3$) using a sterile scalpel. The fragments were washed 2 times with PBS and cultured in 24 well plates in GMP Serum-free DC medium (CellGenix, Freiburg, Germany) plus 5% pooled human AB serum (Innovative Research, Michigan, USA) supplemented with recombinant IL-2 (1000 IU/ml), IL-15 (10 ng/ml), IL-21 (10 ng/ml) (Prospec, Ness-Ziona, Israel). The culture medium was changed when necessary. TILs were transferred into 6 well plates; as they covered >70% of the 24 well surface, they were further expanded in G-Rex flasks (Wilson Wolf, New Brighton, USA) using 30ng OKT3/mL and irradiated allogeneic feeder cells at the ratio of 1 feeder cell to 10 TILs.

#### 1.3 Tumor cell generation

Tumor single cell suspension were processed using a Medimachine (BD, California, USA Cat:340588). The cell suspension was washed 2 times in PBS and then cultured in T25 flasks (Thermo Fisher Scientific Inc., Waltham, MA USA) using RPMI 1640 L-glutamine 2mM (Life Technologies, CA, USA) supplemented with antibiotics plus 20% Fetal Bovine Serum (Gibco, REF: 26140-079).

### Example 2 - Exposure of TIL to autologous tumor cell co-cultures.

It was tested whether the expanded TIL recognized the autologous tumor cell line defined by intracellular cytokine production. For this, expanded TILs were exposed to autologous tumor cells in 96 well plates. Cells were seeded at 20,000 TILs/well at an E:T ratio of 10:1. Supernatants were harvested at day 3 and tested for IFNγ by Elisa (MABTECH, Stockholm, Sweden).

A IFNγ and TNFα production against the autologous tumor cells in expanded TIL from individual patients up to 7.87% in CD3+CD4+ and up to 48.70% in CD3+CD8+ TIL was observed (see Fig. 1)

The cytokine production from GBM-D CD3+CD4+ TCRVβ2 T-cells (representing 33.3% TCRVβ2 T-cells in CD3+CD4 TIL) (Fig. 2): CD3+CD4+ TIL exhibited TNFα production of 3.5% in response to autologous tumor cells, its TCRVβ2 T-cell subpopulation showed a higher frequency (13,6%.) of TNFα producing CD3+CD4+ cells. Testing of TIL reactivity in ICS also revealed selective IL-2 and IL-17 production of individual TIL lines against the autologous tumor cells.

### Example 3 - Link of the individual TCR Vβ families with tumor recognition

TCR Vβ families were analyzed by flow cytometry and PCR.

#### 3.1 Vβ family Analysis by Flow cytometry

TCR Vβ frequency staining was performed using the Beta Mark TCR Vβ Repertoire Kit (Beckman Coulter, CA, USA) along with co-staining with anti-CD3 PE-Cy7 (BD Biosciences, CA, USA), anti-CD4 Krome Orange (Beckman coulter, CA, USA) and anti-CD8a APC-Cy7 (BD Biosciences, CA, USA). After washing, a FACS Aria flow cytometer (BD Biosciences, Stockholm, Sweden) was used for acquisition and data analysis was performed by FlowJo software. TCR CDR3 analysis was performed using the TCR Vβ panel as described before (Magalhaes et al., 2008). In brief, TILs were sorted using CD4+ or CD8+ magnetic beads (MACS Milteny Biotec AB, Lund, Sweden) according to the supplier's instructions.

### 3.2 Vβ family Analysis by PCR

For the PCR analysis, total RNA from CD4+ and CD8+ positive sorted cells was extracted using a RNeasy plus RNA extraction kit (Qiagen inc. Hilden, Germany) and converted to cDNA using a Oligodt protocol from revertaid premium first strand cDNA synthesis kit (Fermentas, ThermoFisher scientific, MA, USA). The multiplex PCR was performed using Qiagen multiplex PCR kit in a 30 μl reaction volume. Each reaction contained 15μl of Qiagen multiplex PCR mastermix, 20pmol of the respective forward primer mix (primer mix 1 to 9), 20 pmol of 6FAM tagged TCRVβ content reverse primer (Ahmed et al., 2014).

A positive control reaction was performed for each sample using primers covering constant region of TCR Vβ gene specific primers tagged with VIC fluorochrome. The PCR was initiated with denaturation at 95 °C for 15 min followed by 35 cycles of 94 °C for 30 sec, 61 °C for 90 sec and 72 °C for 90 sec. The reaction was terminated at 72 °C for 10 min. The analysis was performed on ABI7500 capillary electrophoresis fragment analyzer (Applied Biosystems, CA, USA) using 400 bp internal control ladder for size reference.

Samples exhibiting monoclonal expansion of T-cells were amplified with family specific primers and cloned into a TA (PCR2.1 topo) vector (Invitrogen, CA, USA) according to the suppliers instructions. The inserts in the plasmids were sequenced to identify the TCR CDR3 region using BigDye terminator cycle sequencing kit and ABI7500 capillary electrophoresis instrument (Applied Biosystems, CA, USA).

### 3.2 Results

The analyzed T-cell products showed up to 99% of individual Vβ families in TIL from individual patients **(see Table 3),** e.g. the TCRVβ2 which represented 99,6 % in CD3+CD4+ T-cells from the GBM-I TIL, the TCRVβ.1 family constituted 97% of GBM-I CD3+CD8+ T-cells; TCRVβ21,3 constituted 98,4% of the GBM-J CD3+CD4+ T-cells. Some of the dominant TCR Vβ families were analyzed in greater detail and tested for the CDR3 length, followed by sequencing of the TCR, i.e. some of the expanded TCRVβ (see **Table 4)** represent T-cell clones.

## Table 3: TCR Vβ CDR3 analysis

| Patient | GBM-A | | GBM-B | | GBM-C | | GBM-D | | GBM-E | | GBM-F | | GBM-G | | GBM-H | | GBM-I | |
|---|---|---|---|---|---|---|---|---|---|---|---|---|---|---|---|---|---|---|
| | CD4 | CD8 | CD4 | CD8 | CD4 | CD8 | CD4 | CD8 | CD4 | CD8 | CD4 | CD8 | CD4 | CD8 | *CD4 | CD8 | CD4 | CD8 |
| Vβ1 | 0,24 | 0,74 | 2,45 | 0,74 | 1,49 | 11,00 | 0,19 | 0,12 | 4,06 | 0,25 | 0,09 | 10,30 | 0,00 | 0,00 | 0,19 (2) | 0,06 | 0,01 | 0,00 |
| Vβ2 | 34,3 (1) | 0,11 | 17,60 | 2,52 | 7,92 | 3,69 | 33,3 (O) | 1,67 | 13,60 | 2,48 | 0,17 | 0,00 | 0,54 | 0,00 | 1,21 (1) | 0,00 | 99,6 (2) | 0,00 |
| Vβ3 | 3,54 | 0,36 | 2,97 | 9,29 | 6,83 | 10,30 | 1,33 | 1,00 | 9,81 | 2,11 | 9,21 | 0,00 | 0,00 | 0,00 | 0,92 | 0,00 | 0,00 | 0,00 |
| Vβ4 | 0,14 | 0,03 | 0,27 | 0,04 | 0,04 | 0,01 | 0,05 | 0,07 | 0,07 | 0,03 | 0,34 | 0,00 | 0,00 | 0,00 | 0,16 | 0,09 | 0,08 | 0,00 |
| Vβ5.1 | 0,34 | 0,18 | 1,72 | 0,76 | 0,18 | 0,11 | 0,14 | 0,09 | 9,98 | 0,11 | 0,06 | 0,00 | 0,00 | 0,00 | 0,24 | 0,00 | 0,00 | 97 (2) |
| Vβ5.2 | 1,63 | 0,10 | 0,65 | 0,33 | 0,65 | 0,87 | 0,23 | 2,23 | 0,40 | 0,16 | 0,56 | 0,00 | 0,07 | 0,00 | 0,71 | 0,25 | 0,04 | 0,00 |
| Vβ5.3 | 0,07 | 0,23 | 1,01 | 0,82 | 0,42 | 1,48 | 0,03 | 0,08 | 0,64 | 0,02 | 0,05 | 0,00 | 0,01 | 0,00 | 0,08 | 0,00 | 0,00 | 0,00 |
| Vβ7.1 | 1,91 | 0,11 | 0,86 | 0,38 | 2,00 | 4,99 | 0,07 | 1,80 | 1,28 | 0,03 | 0,08 | 0,00 | 0,00 | 0,00 | 0,64 | 0,00 | 0,01 | 0,00 |
| Vβ7.2 | 3,73 | 1,53 | 1,53 | 0,86 | 1,19 | 0,32 | 0,35 | 6,80 | 0,97 | 0,03 | 0,13 | 0,00 | 0,00 | 0,00 | 0,00 | 0,00 | 0,09 | 0,00 |
| Vβ8 | 2,21 | 1,67 | 6,90 | 3,25 | 4,89 | 1,23 | 2,34 | 1,23 | 6,71 | 0,25 | 1,10 | 0,00 | 0,03 | 0,00 | 4,77 (1) | 40,4 (2) | 0,01 | 0,65 |
| Vβ9 | 0,90 | 0,01 | 0,56 | 0,53 | 2,74 | 0,14 | 0,28 | 1,00 | 0,18 | 5,66 | 0,04 | 0,00 | 0,04 | 2,86 | 0,39 | 0,11 | 0,00 | 0,27 |
| Vβ11 | 0,25 | 0,15 | 0,12 | 2,17 | 0,95 | 2,64 | 0,43 | 0,42 | 0,85 | 0,05 | 0,02 | 0,00 | 0,01 | 0,00 | 0,19 | 0,07 | 0,07 | 0,00 |
| Vβ12 | 2,37 | 28,4 (O) | 4,18 | 2,34 | 4,44 | 2,20 | 3,67 | 0,46 | 12,10 | 1,04 | 0,03 | 0,00 | 0,02 | 0,00 | 0,14 | 0,00 | 0,06 | 0,00 |
| Vβ13.1 | 0,88 | 3,26 | 2,60 | 1,12 | 5,27 | 1,14 | 4,47 | 2,08 | 0,41 | 0,06 | 0,02 | 0,00 | 85,1 (1) | 0,00 | 1,08 | 0,08 | 0,00 | 0,00 |
| Vβ13.2 | 0,35 | 0,03 | 3,25 | 2,02 | 1,06 | 2,95 | 0,88 | 0,56 | 0,59 | 0,10 | 0,71 | 8,70 | 0,10 | 3,57 | 0,08 | 0,27 | 0,01 | 0,00 |
| Vβ13.6 | 2,14 | 0,19 | 1,64 | 0,15 | 1,84 | 0,60 | 0,56 | 0,74 | 3,01 | 0,05 | 0,12 | 0,00 | 0,37 | 0,00 | 0,32 | 0,08 | 0,00 | 0,00 |
| Vβ14 | 0,76 | 4,47 | 3,63 | 3,87 | 6,75 | 6,03 | 2,00 | 3,38 | 3,87 | 1,98 | 48,2 (O) | 5,26 | 0,01 | 0,00 | 1,68 | 45,3 (2) | 0,01 | 0,00 |
| Vβ16 | 2,90 | 0,70 | 3,84 | 1,97 | 4,28 | 5,27 | 17,7 (N) | 4,38 | 2,27 | 1,70 | 0,24 | 0,00 | 0,00 | 0,00 | 0,21 | 0,05 | 0,01 | 0,00 |
| Vβ17 | 0,35 | 0,03 | 1,35 | 0,28 | 4,71 | 0,00 | 0,11 | 1,25 | 0,83 | 0,27 | 0,14 | 0,00 | 0,00 | 42,9 (N) | 10,20 | 0,00 | 0,02 | 0,00 |
| Vβ18 | 0,08 | 0,08 | 0,62 | 0,10 | 0,21 | 0,08 | 0,39 | 0,04 | 0,80 | 0,03 | 0,01 | 0,00 | 0,01 | 0,00 | 0,00 | 0,00 | 0,00 | 0,00 |

| | | | | | | | | | | | | | | | | | | |
|---|---|---|---|---|---|---|---|---|---|---|---|---|---|---|---|---|---|---|
| **Vβ20** | 16,7 (N) | 0,45 | 10,50 | 2,92 | 8,16 | 4,39 | 3,67 | 2,88 | 2,68 | 0,31 | 0,08 | 0,00 | 8,48 | 2,04 (1) | 3,62 | 0,06 | 0,00 | 0,43 |
| **Vβ21.3** | 0,41 | 41,5 (2) | 2,97 | 3,71 | 3,61 | 8,54 | 4,18 | 6,71 | 2,47 | 45,90 | 3,34 | 3,45 | 0,01 | 0,00 | 0,13 | 0,50 | 0,01 | 0,00 |
| **Vβ22** | 26,80 | 0,06 | 0,70 | 0,00 | 1,63 | 1,63 | 1,33 | 35,4 (O) | 6,50 | 0,02 | 0,96 | 0,00 | 0,01 | 0,00 | 0,46 (1) | 9,96 | 0,01 | 0,00 |
| **Vβ23** | 1,39 | 0,07 | 0,79 | 0,53 | 1,20 | 2,56 | 0,10 | 0,09 | 0,91 | 0,02 | 0,05 | 0,00 | 0,12 | 0,00 | 5,42 | 0,19 | 0,00 | 0,00 |

| Patient | GBM-J | GBM-K | | GBM-L | | GBM-M | | GBM-N | | GBM-0 | | GBM-P | |
|---|---|---|---|---|---|---|---|---|---|---|---|---|---|
| | CD4 | *CD4 | *CD8 | CD4 | CD8 | *CD4 | CD8 | CD4 | CD8 | CD4 | CD8 | CD4 | CD8 |
| **Vβ1** | 0,02 | 0,58 (1) | 0 (1) | 1,72 | 1,78 | 0,47 | 1,72 | 0,27 | 0,28 | 1,37 | 3,70 | 0,13 | 0,04 |
| **Vβ2** | 0,06 | 0,25 (1) | 0,029 (1) | 12,20 | 0,90 | 1,39 | 3,72 | 40,90 | 0,04 | 2,38 | 2,30 | 1,24 | 0,02 |
| **Vβ3** | 0,00 | 0,81 (1) | 0,12 (1) | 4,46 | 0,38 | 19,30 | 3,70 | 1,33 | 0,02 | 1,34 | 3,09 | 0,12 | 0,20 |
| **Vβ4** | 0,00 | 0,11 | 0,00 | 0,47 | 0,08 | 0,15 | 0,98 | 0,03 | 0,01 | 0,01 | 0,08 | 0,01 | 0,01 |
| **Vβ5.1** | 0,01 | 0,39 | 0,37 | 7,32 | 1,67 | 0,08 | 0,00 | 0,33 | 0,45 | 0,16 | 0,26 | 0,29 | 0,18 |
| **Vβ5.2** | 0,32 | 0,43 | 0,06 | 0,47 | 0,61 | 0,15 | 0,53 | 1,28 | 0,10 | 0,27 | 0,80 | 0,26 | 0,18 |
| **Vβ5.3** | 0,00 | 0,09 | 0,00 | 2,91 | 4,48 | 0,22 | 0,85 | 0,05 | 0,02 | 0,06 | 0,13 | 0,06 | 1,37 |
| **Vβ7.1** | 0,00 | 0,21 | 0,00 | 1,12 | 1,51 | 0,65 | 1,99 | 5,07 | 90,70 | 0,05 | 0,25 | 0,15 | 0,37 |
| **Vβ7.2** | 0,01 | 0,97 | 0,04 | 2,71 | 6,92 | 0,05 | 2,93 | 0,11 | 0,00 | 0,95 | 1,39 | 0,01 | 0,04 |
| **Vβ8** | 0,00 | 3,02 | 2,65 | 4,88 | 2,35 | 3,41 | 23,10 | 1,02 | 0,07 | 0,28 | 0,22 | 0,13 | 0,23 |
| **Vβ9** | 0,00 | 0,02 | 0,00 | 2,19 | 0,72 | 1,70 | 3,55 | 7,62 | 0,07 | 0,31 | 2,97 | 7,98 | 0,16 |
| **Vβ11** | 0,01 | 0,29 | 0,04 | 2,82 | 0,69 | 0,19 | 0,43 | 0,10 | 0,10 | 0,13 | 0,30 | 0,21 | 1,63 |
| **Vβ12** | 0,00 | 0,53 | 0,00 | 2,60 | 0,34 | 1,93 | 6,91 | 1,81 | 0,04 | 0,51 | 11,60 | 0,09 | 0,02 |
| **Vβ13.1** | 0,03 | 0,09 | 0,06 | 15,50 | 0,62 | 11,10 | 1,54 | 0,18 | 0,05 | 3,15 | 2,44 | 23,90 | 0,03 |
| **Vβ13.2** | 0,01 | 6,41 | 0,38 | 3,14 | 6,70 | 19,90 | 1,95 | 0,87 | 0,27 | 0,18 | 2,56 | 0,41 | 1,58 |
| **Vβ13.6** | 0,00 | 0,16 | 0,15 | 2,71 | 6,54 | 1,08 | 3,08 | 0,09 | 0,27 | 0,15 | 0,41 | 1,34 | 0,03 |

| Vβ | | | | | | | | | | | | | |
|---|---|---|---|---|---|---|---|---|---|---|---|---|---|
| **Vβ14** | 0,28 | 0,57 | 2,84 | 2,48 | 0,01 | 0,13 | 18,70 | 1,91 | 1,80 | 2,72 | 0,77 | 13,7 (N) | 0,00 |
| **Vβ16** | 0,17 | 1,33 | 1,92 | 0,23 | 0,04 | 0,16 | 5,08 | 0,97 | 4,17 | 5,29 | 0,06 | 1,59 | 0,02 |
| **Vβ17** | 0,07 | 56,00 | 21,80 | 0,06 | 0,06 | 0,40 | 1,52 | 0,83 | 1,94 | 2,91 | 0,03 | 0,06 | 0,00 |
| **Vβ18** | 0,15 | 0,24 | 0,26 | 0,12 | 0,05 | 0,03 | 0,00 | 0,01 | 7,08 | 3,17 | 0,09 | 0,34 | 0,00 |
| **Vβ20** | 0,05 | 0,18 | 1,15 | 0,12 | 0,01 | 0,36 | 0,00 | 0,10 | 1,32 | 4,84 | 0,46 (1) | 1,1 (2) | 1,01 |
| **Vβ21.3** | 0,07 | 9,50 | 1,06 | 0,20 | 0,06 | 0,08 | 23,30 | 0,23 | 36,40 | 1,15 | 0,04 | 5,06 | 98,4 (2) |
| **Vβ22** | 7,30 | 0,06 | 0,40 | 0,22 | 0,11 | 0,04 | 0,00 | 0,30 | 1,09 | 1,13 | 0,21 | 1,01 | 0,10 |
| **Vβ23** | 0,16 | 0,37 | 5,83 | 0,83 | 0,58 | 0,40 | 0,86 | 0,36 | 3,82 | 2,86 | 0,20 | 1,39 | 0,00 |

Table 4: Vβ amino acid sequences

| TIL id | Vβ | Vβ | (-D-) | Jβ | Jβ | Flow antibody |
|---|---|---|---|---|---|---|
| **GBM A-CD4** | TRBV20-1 | CSA | ATGDRP | YEQYF | TRBJ2-7 | Vβ2 |
| **GBM A-CD8** | TRBV10-3 | CAI | RTGSD | NEQSF | TRBJ2-1 | Vβ12 |
| **GBM A-CD8** | TRV11 | CAS | RYTGS | IEQFF | TRBJ2-1 | Vβ21.3 |
| **GBM F-CD4** | TRBV27 | CAS | SAGTSGVT | YEQYF | TRBJ2-7 | Vβ14 |
| **GBM G-CD4** | TRBV6-1/5/6 | CAS | STR | FEQYF | TRBJ2-7 | Vβ13.1 |
| **GBM H-CD8** | TRBV12-3/4 | CAS | SSRRDHTY | NGQFF | TRBJ2-1 | Vβ8 |

(continued)

| TIL id | Vβ | Vβ | (-D-) | Jβ | Jβ | Flow antibody |
|--------|------|------|----------------|-------|----------|---------------|
| **GBM H-CD8** | TRV27 | CAS | SLQGAN | YGYTF | TRBJ1-2 | Vβ14 |
| **GBM I-CD4** | TRBV20-1 | CSA | RVIPSGGV VQGT | DTQYF | TRBJ2-3 | Vβ2 |
| **GBM I-CD8** | TRBV5-1 | CAS | SWDKS | YEQYF | TRBJ2-7 | Vβ5.1 |
| **GBM J-CD4** | TRBV11 | CAS | SRLALFS | YEQYF | TRBJ2-7 | Vβ21.3 |

**Example 4 - Expansion of TIL and tumor cell culture from pancreas cancer patients**

4.1 Diagnosis and Patients.

17 patients (Table 1) with pancreatic cancer (13/17 with a ductal adenocarcinoma), recruited at the Pancreatic Surgery Unit of the Karolinska Hospital, Huddinge, Sweden, provided informed consent. The study was approved by the regional ethical review board at Karolinska Institutet, Stockholm, Sweden (Dnr: 2013/576-31).

4.2 Initial TIL culture

Tumor tissues from clinical biopsies or surgery were cut with surgical scissors and a scalpel. Individual single tumor fragments (1-2 mm$^3$) were placed in each well of a 24-well tissue culture plate (Costar, USA) along with 1 ml of TIL medium , i.e. Cellgro GMP Serum-free medium (CellGenix, Freiburg, Germany), with 10% human AB serum (Innovative Research, Michigan, USA), supplemented with IL-2 1000IU/ml, IL-15 10ng/ml, IL-21 10ng/ml, (Prospec, Ness-Ziona, Israel), penicillin (100IU/mL), streptomycin (100·g/mL) (Life Technologies, Carlsbad, USA) and Amphotericin B (2.5mg/L) (Sigma-Aldrich, MI; USA). Plates were then incubated at 37°C, 5% CO2. On day 4, 1 ml medium was added to wells. On day 7, TIL were split into 2-3 additional 24-wells. Upon 80% expansion of TIL in each culture well, 1 ml of TIL medium was added.

4.3 TIL Expansion Protocol.

TIL were further expanded on day 10 using OKT3 (Biolegend, CA, USA) in the presence of a 1:10 ratio of 55 Gry irradiated allogeneic feeder cells from healthy volunteers, resuspended in medium (Cellgro with 10% human AB serum). OKT3 was used at 30 ng/ml along with IL-2 1000IU/ml, IL-15 10ng/ml, and IL-21 10ng/ml. TIL were then transferred to 6-well plates and tested for anti-tumor (as autologous tumor cells were available) or TAA-reactivity. If tested positive, defined by IFN-production, they were expanded in GRex flasks (Wilson Wolf, New Brighton, MN, USA) using TIL medium (Cellgro with 10% human AB serum, IL-2 1000IU/ml, IL-15 10ng/ml, and IL-21 10ng/m.) plus irradiated (55Gy) feeder cells (ratio of feeder cells: TIL was 1:10); OKT3 was used at 30 ng/ml.

4.4 Tumor cell generation

After surgery or biopsy, tumor tissues were cut with surgical scissors and a scalpel. Single tumor fragments (1-2 mm3) were placed in 24-well tissue culture plates with 1 ml of tumor medium, i.e. RPMI 1640 with 10% FBS (Life technologies, CA, USA), Epidermal growth factor (20ng/ml, ImmunoTools, Friesoythe, Germany) supplemented with antibiotics (penicillin,100IU/mL and streptomycin, 10mg/mL) (Life Technologies, Carlsbad, USA) and Amphotericin B (2.5mg/L, Sigma-Aldrich, MI; USA). Tumor cell lines were then cultured and passed without EGF.

**Example 5 - Pancreas cancer: Exposure of TIL to autologous tumor cell co-cultures.**

The autologous tumor cell line established from Panc 17 was tested for TIL recognition **(Table 5 )**. IL-2 and IL-17 production tested positive in CD3+CD4+ TIL (1.55%). CD3+CD4-CD8- (DN) T-cells showed IL-2 (3.71%) and IL-17 (9.76%) production in response to autologous tumor cells. The same TIL line showed strong cytotoxicity directed against tumor cells, tested by a standard Cr51 release assay (see **Figure** 3A).

The Panc 9 TIL (99.2% CD8+ Vβ13.2, a monoclonal TCR see supplementary Table S2) exhibited IFN-y production against freshly harvested autologous tumor single cell suspension cells, i.e. 2022.81 pg/ml (see Figure 3B), which could be blocked using the anti-MHC-I antibody (W6/32, 9.91 pg/ml); the anti-HLA-DR antibody (L243) did not affect IFN-y production (1853.45pg/ml).

**Example 6 TCRVβ CDR3 analysis**

We analyzed the TCR Vβ families in TIL with a preferential expansion of individual Vβ families.

6.1 TCRVβ CDR3 analysis by Flow cytometry

TCR Vβ frequency staining was performed using the Beta Mark TCR Vβ Repertoire Kit (Beckman Coulter, France) along with CD3 PE-Cy7 (BD Biosciences, CA, USA), CD4 Krome orange (Beckman Coulter, France), and CD8 APC-Cy7(BD Biosciences, CA, USA). TIL were washed using PBS-0.1 %FBS and stained using a FACS Aria flow cytometer (BD Biosciences, Stockholm, Sweden).

6.2 TCRVβ CDR3 analysis by PCR:

TCR CDR3 analysis was performed using the TCR Vβ panel as described before [29]. The TCR Vβ CDR3 analysis was performed using the TCR Vβ family specific primer set panel previously described [30]. First, TILs were sorted using CD4+ or CD8+ magnetic beads (MACS Miltenyibiotec AB, Lund, Sweden) according to the supplier's instructions. Total RNA from CD4 and CD8 positive sorted cells was extracted using a RNeasy plus RNA extraction kit (Qiagen inc. Hilden, Germany) and converted to cDNA using a Oligodt protocol from the revertaid premium first strand cDNA synthesis kit (Fermentas, ThermoFisher scientific, MA, USA). The multiplex PCR was performed using Qiagen multiplex PCR kit in a 30· I reaction volume. Each reaction contained 15· I of the Qiagen multiplex PCR mastermix, 20pmol of the respective forward primer mix (primer mix 1 to 9) and 20pmol of 6FAM tagged TCRVβ content reverse primer. A positive control reaction was performed for each sample using primers covering constant region of TCR Vβ gene specific primers tagged with VIC fluorochrome. The PCR was initiated with denaturation at 95°C for 15 min followed by 35 cycles of 94°C for 30 seconds, 61°C for 1 minute 30 seconds and 72°C for 1 minute 30 seconds. The reaction was terminated at 72°C for 10 minutes. Analysis was performed on ABI7500 capillary electrophoresis fragment analyzer (Applied Biosystems, CA, USA) using an 400bp internal control ladder for size reference. Samples exhibiting strong monoclonal expansion of T-cells were amplified with family specific primers and cloned into a TA (PCR2.1 topo) vector (Invitrogen, CA, USA) according to the suppliers instructions. The inserts in the plasmids were sequenced to identify the TCR CDR3 region using BigDye terminator cycle sequencing kit and the ABI7500 capillary electrophoresis instrument (Applied Biosystems, CA, USA).

6.3 Results

Panc 9 (CD8+) TIL exhibited up to 99.2% Vβ13.2 + T-cells. The preferential expansion of TCR Vβ families was also observed in other TIL lines **(see Table 5),** e.g. CD8+ Vβ1 (77.1 %) from Panc1. CD4+ Vβ8 (42.7 %) in Panc5, CD8+ Vβ2 (39.8 %) in Panc10, CD4+ Vβ2 (48.9 %) in Panc16. Some of the expanded Vβ families showed clonal TCRs tested by CDR3 TCR length analysis, followed by TCR sequencing (see **Tables 6 & 7** and **Fig. 4).**

Table 5: Vβ analysis in TILs. Numbers indicated the frequency of TCR Vbeta families in CD4+ and CD8+ TIL

|  | Panc 1 | | Panc 2 | | Panc 3 | | Panc 4 | | Panc 5 | | Panc 6 | | Panc 7 | | Panc 8 | | Panc 9 | |
|---|---|---|---|---|---|---|---|---|---|---|---|---|---|---|---|---|---|---|
|  | CD4 | CD8 | CD4 | CD8 | CD4 | CD8 | CD4 | CD8 | CD4 | CD8 | CD4 | CD8 | CD4 | CD8 | CD4 | CD8 | CD4 | CD8 |
| Vβ1 | 2.8 | 77.1 | 3.2 | 2.3 | 3.4 | 0.8 | 0.2 | 2.1 | 1.5 | 0.2 | 0.7 | 1.1 | 0.3 | 0.1 | 1.5 | 1.2 | 0.6 | 0.1 |
| Vβ2 | 8.0 | 0.6 | 9.8 | 2.6 | 4.8 | 0.2 | 8.5 | 2.0 | 0.0 | 0.0 | 9.6 | 19.9 | 0.1 | 0.7 | 7.4 | 1.7 | 12.0 | 0.0 |
| Vβ3 | 2.1 | 0.2 | 4.8 | 1.9 | 26.7 | 0.5 | 4.8 | 8.3 | 0.0 | 6.1 | 4.2 | 0.3 | 5.1 | 28.7 | 2.4 | 12.0 | 1.0 | 0.0 |
| Vβ4 | 0.4 | 0.0 | 0.0 | 0.0 | 0.1 | 0.0 | 0.0 | 0.0 | 0.0 | 0.0 | 0.0 | 0.0 | 0.1 | 0.0 | 0.1 | 0.0 | 3.2 | 0.0 |
| Vβ5.1 | 0.4 | 0.4 | 0.7 | 0.5 | 0.3 | 0.0 | 0.1 | 0.0 | 0.0 | 0.3 | 9.0 | 0.3 | 41.1 | 6.2 | 9.3 | 0.0 | 3.6 | 0.1 |
| Vβ5.2 | 0.2 | 0.1 | 1.5 | 0.0 | 1.5 | 0.0 | 0.1 | 0.2 | 0.2 | 0.0 | 0.2 | 13.7 | 2.4 | 0.2 | 0.3 | 0.2 | 0.1 | 0.1 |
| Vβ5.3 | 0.1 | 0.1 | 1.6 | 0.4 | 1.9 | 0.0 | 0.0 | 0.0 | 0.0 | 0.0 | 0.3 | 0.1 | 0.0 | 0.9 | 0.6 | 0.1 | 0.0 | 0.0 |
| Vβ7.1 | 0.2 | 0.3 | 0.7 | 2.1 | 0.9 | 3.4 | 0.2 | 0.1 | 0.1 | 0.0 | 0.1 | 0.1 | 0.1 | 0.2 | 5.1 | 0.3 | 0.6 | 0.0 |
| Vβ7.2 | 6.6 | 0.0 | 0.3 | 0.1 | 0.9 | 1.7 | 0.2 | 0.1 | 0.0 | 0.0 | 1.1 | 0.1 | 1.0 | 0.4 | 5.3 | 3.8 | 13.9 | 0.0 |
| Vβ8 | 1.9 | 1.7 | 3.7 | 0.6 | 2.9 | 0.1 | 0.4 | 0.4 | 42.7 | 4.1 | 1.2 | 0.3 | 5.4 | 0.5 | 2.3 | 2.2 | 0.5 | 0.1 |
| Vβ9 | 1.6 | 0.0 | 0.8 | 0.3 | 0.3 | 0.1 | 0.1 | 0.1 | 40.5 | 0.1 | 1.7 | 0.0 | 0.2 | 0.1 | 1.0 | 0.1 | 4.8 | 0.0 |
| Vβ11 | 0.5 | 0.0 | 0.5 | 0.2 | 1.5 | 0.0 | 0.1 | 0.7 | 0.3 | 0.0 | 0.6 | 2.3 | 4.0 | 1.0 | 0.2 | 0.0 | 1.0 | 0.1 |
| Vβ12 | 3.2 | 0.9 | 1.9 | 0.9 | 2.5 | 0.1 | 0.7 | 1.8 | 0.0 | 0.0 | 0.6 | 0.3 | 0.0 | 0.0 | 0.1 | 0.1 | 0.4 | 0.0 |
| Vβ13.1 | 2.4 | 0.3 | 4.6 | 10.4 | 1.5 | 0.5 | 2.6 | 4.2 | 0.0 | 0.1 | 0.8 | 0.0 | 8.1 | 10.0 | 5.6 | 16.5 | 5.0 | 0.0 |
| Vβ13.2 | 3.1 | 0.2 | 1.4 | 0.4 | 1.8 | 0.2 | 0.2 | 0.1 | 0.0 | 0.0 | 0.2 | 0.6 | 0.3 | 1.1 | 0.3 | 0.0 | 0.5 | 99.2 |
| Vβ13.6 | 2.8 | 9.1 | 3.6 | 0.5 | 1.2 | 0.1 | 0.2 | 0.1 | 0.2 | 0.1 | 0.2 | 0.0 | 1.7 | 0.1 | 3.2 | 6.9 | 0.4 | 0.0 |
| Vβ14 | 1.9 | 1.4 | 2.0 | 3.6 | 1.3 | 63.3 | 0.4 | 0.7 | 0.0 | 0.0 | 1.0 | 5.3 | 3.2 | 2.8 | 0.6 | 1.4 | 0.4 | 0.1 |
| Vβ16 | 2.4 | 1.0 | 6.9 | 6.9 | 3.3 | 0.3 | 4.1 | 1.1 | 0.0 | 0.1 | 0.8 | 0.0 | 0.1 | 0.3 | 0.1 | 25.4 | 0.5 | 0.0 |
| Vβ17 | 4.6 | 0.1 | 2.9 | 1.8 | 0.8 | 0.0 | 0.2 | 0.0 | 0.0 | 0.0 | 1.5 | 0.0 | 1.0 | 32.9 | 1.3 | 0.1 | 12.4 | 0.0 |
| Vβ18 | 2.4 | 0.1 | 0.2 | 0.0 | 0.5 | 0.0 | 0.1 | 0.0 | 0.4 | 0.0 | 0.4 | 0.2 | 1.0 | 0.2 | 3.6 | 0.0 | 0.4 | 0.0 |
| Vβ20 | 7.0 | 5.9 | 6.6 | 13.8 | 4.6 | 0.1 | 3.7 | 0.1 | 0.0 | 0.4 | 0.7 | 0.2 | 0.1 | 0.0 | 1.4 | 0.4 | 0.1 | 0.0 |
| Vβ21.3 | 0.4 | 0.5 | 3.8 | 4.3 | 0.4 | 0.1 | 0.1 | 0.1 | 0.0 | 3.2 | 6.2 | 0.1 | 2.4 | 0.0 | 0.6 | 0.3 | 0.5 | 0.0 |
| Vβ22 | 11.1 | 0.2 | 3.3 | 1.7 | 2.8 | 0.2 | 6.2 | 1.8 | 0.1 | 0.0 | 15.1 | 2.1 | 0.2 | 2.6 | 1.4 | 0.6 | 1.9 | 0.7 |
| Vβ23 | 3.7 | 0.3 | 0.4 | 0.1 | 0.8 | 0.8 | 0.9 | 0.5 | 1.6 | 0.5 | 0.1 | 4.6 | 0.1 | 0.1 | 0.2 | 6.7 | 0.1 | 0.0 |

Table 5 (continued)

| | Panc 10 | | Panc 11 | | Panc 12 | | Panc 13 | | Panc 14 | | Panc 15 | | Panc 16 | | Panc 17 | |
|---|---|---|---|---|---|---|---|---|---|---|---|---|---|---|---|---|
| | CD4 | CD8 | CD4 | CD8 | CD4 | CD8 | CD4 | CD8 | CD4 | CD8 | CD4 | CD8 | CD4 | CD8 | CD4 | CD8 |
| Vβ1 | 4.4 | 7.1 | 13.0 | 0.5 | 1.4 | 25.8 | 0.1 | 0.3 | 0.9 | 16.2 | 0.6 | 2.8 | 0.3 | 0.4 | 2.2 | 5.7 |
| Vβ2 | 4.1 | 39.8 | 2.4 | 0.2 | 7.5 | 4.8 | 1.5 | 0.6 | 5.1 | 0.7 | 29.3 | 1.5 | 48.9 | 1.0 | 16.3 | 1.1 |
| Vβ3 | 3.7 | 1.3 | 0.0 | 0.0 | 1.7 | 8.8 | 0.0 | 0.0 | 13.3 | 2.0 | 1.1 | 17.9 | 11.7 | 0.2 | 17.8 | 2.6 |
| Vβ4 | 6.0 | 0.1 | 0.0 | 0.0 | 2.2 | 0.4 | 4.8 | 0.0 | 0.9 | 0.1 | 8.3 | 1.2 | 0.1 | 0.1 | 0.0 | 0.0 |
| Vβ5.1 | 10.8 | 1.1 | 0.6 | 0.7 | 3.4 | 0.5 | 4.6 | 0.2 | 2.7 | 0.8 | 4.2 | 1.6 | 15.2 | 0.2 | 7.7 | 0.0 |
| Vβ5.2 | 1.3 | 9.4 | 0.1 | 0.1 | 0.3 | 1.8 | 0.1 | 0.1 | 0.4 | 0.1 | 2.3 | 0.8 | 1.2 | 0.1 | 0.4 | 0.0 |
| Vβ5.3 | 0.7 | 18.0 | 0.2 | 0.0 | 0.3 | 1.6 | 0.1 | 0.0 | 0.1 | 0.3 | 4.2 | 0.3 | 0.9 | 0.0 | 1.4 | 0.4 |
| Vβ7.1 | 0.5 | 1.5 | 0.0 | 0.0 | 1.6 | 2.8 | 0.1 | 0.3 | 0.3 | 6.3 | 0.9 | 1.8 | 0.3 | 2.2 | 1.1 | 1.5 |
| Vβ7.2 | 1.6 | 0.1 | 0.0 | 0.0 | 0.4 | 0.1 | 0.0 | 0.0 | 0.7 | 4.8 | 0.4 | 0.1 | 0.0 | 0.0 | 2.2 | 1,4 |
| Vβ8 | 2,7 | 1,4 | 0,4 | 0,3 | 3,6 | 1,8 | 1,1 | 0,5 | 1,7 | 0,8 | 1,9 | 4,1 | 0,5 | 0,3 | 2,7 | 1,9 |
| Vβ9 | 3,4 | 1,2 | 0,5 | 0,2 | 6,7 | 1,0 | 2,4 | 0,1 | 9,7 | 2,3 | 2,0 | 2,0 | 1,8 | 0,4 | 1,9 | 1,5 |
| Vβ11 | 0,6 | 0,3 | 0,1 | 0,2 | 0,8 | 0,3 | 0,2 | 0,2 | 0,3 | 0,2 | 1,4 | 0,7 | 0,4 | 0,2 | 0,2 | 0,0 |
| Vβ12 | 1,6 | 0,5 | 0,0 | 0,0 | 0,3 | 1,0 | 0,3 | 0,0 | 1,5 | 5,9 | 0,3 | 0,2 | 0,3 | 0,1 | 4,8 | 0,7 |
| Vβ13.1 | 3,7 | 0,1 | 35,5 | 0,1 | 9,2 | 0,3 | 0,5 | 0,2 | 4,3 | 19,6 | 0,7 | 0,8 | 2,6 | 1,1 | 1,5 | 0,9 |
| Vβ13.2 | 6,2 | 0,7 | 0,2 | 0,0 | 2,5 | 1,4 | 0,2 | 0,2 | 4,4 | 0,9 | 1,8 | 23,0 | 0,4 | 0,6 | 3,2 | 3,4 |
| Vβ13.6 | 2,0 | 0,3 | 0,6 | 0,0 | 1,5 | 0,2 | 0,5 | 0,2 | 0,3 | 1,9 | 0,6 | 0,9 | 0,3 | 0,4 | 1,1 | 2,0 |
| Vβ14 | 3,6 | 0,8 | 0,1 | 0,0 | 0,7 | 3,5 | 0,1 | 0,3 | 2,9 | 2,5 | 0,9 | 7,2 | 2,1 | 0,6 | 1,6 | 7,6 |
| Vβ16 | 1,0 | 0,2 | 0,0 | 0,2 | 0,1 | 0,1 | 0,0 | 0,2 | 0,2 | 0,8 | 0,2 | 0,2 | 0,1 | 0,0 | 1,3 | 0,0 |
| Vβ17 | 7,5 | 0,3 | 0,1 | 12,3 | 3,3 | 2,3 | 31,1 | 0,9 | 9,9 | 1,4 | 1,3 | 1,9 | 1,5 | 0,5 | 0,3 | 0,1 |
| Vβ18 | 0,5 | 0,2 | 0,1 | 0,2 | 0,3 | 0,3 | 0,5 | 0,1 | 0,9 | 3,1 | 3,3 | 3,6 | 0,4 | 3,3 | 0,3 | 0,0 |
| Vβ20 | 2,5 | 0,5 | 0,1 | 0,1 | 1,4 | 0,2 | 0,0 | 0,0 | 0,7 | 0,3 | 0,5 | 0,6 | 0,0 | 0,8 | 1,3 | 4,0 |
| Vβ21.3 | 2,4 | 0,1 | 0,9 | 0,0 | 0,5 | 8,0 | 0,5 | 0,0 | 1,2 | 0,5 | 0,4 | 0,4 | 0,2 | 1,6 | 1,5 | 6,8 |
| Vβ22 | 3,4 | 1,0 | 3,6 | 0,1 | 5,2 | 2,0 | 26,0 | 65,9 | 3,6 | 0,8 | 0,7 | 2,9 | 6,2 | 6,4 | 2,6 | 0,2 |
| Vβ23 | 4,5 | 0,7 | 0,6 | 0,1 | 0,2 | 0,4 | 0,3 | 1,0 | 0,5 | 0,7 | 1,8 | 7,4 | 0,3 | 0,3 | 1,1 | 2,0 |

**Table 6:** Vβ analysis and clonality in TILs after expansion

| Patient | Cell population | Family by per | Vβ by cytometry | Flow Number | of clones |
|---------|-----------------|---------------|-----------------|-------------|-----------|
| Panc 1 | CD8 | TRBV9 | Vβ1 | 2 | |
| Panc 5 | CD4 | TRBV12 | Vβ8 | 1 | |
| Panc 7 | CD4 | TRBV5,1 | Vβ5,1 | 1 | |
| | CD8 | TRBV19 | Vβ17 | 2 | |
| | | TRBV28 | Vβ3 | 2 | |
| Panc 8 | CD8 | TRBV14 | Vβ16 | 1 | |
| Panc 9 | CD8 | TRBV6 | Vβ13,2 | 1 | |
| Panc 10 | CD8 | TRBV20,1 | Vβ2 | 2 | |
| Panc 11 | CD8 | TRBV20 | Vβ2 | **1** | |
| | | TRBV7,3/6/7/9 | No antibody | 1 | |
| | | TRBV19 | Vβ17 | 1 | |
| Panc 13 | CD4 | TRBV19 | Vβ17 | 1 | |
| Panc 14 | CD8 | TRBV9 | Vβ1 | 2 | |
| Panc 15 | CD4 | TRBV20,1 | Vβ2 | 2 | |
| | CD8 | TRBV6,2 | Vβ13,2 | 2 | |
| Panc 16 | CD4 | TRBV20,1 | Vβ2 | 1 | |
| | CD8 | TRBV7,3 | No antibody | 2 | |
| | | TRBV7,2,8 | No antibody | 2 | |
| | | TRBV19 | Vβ17 | 1 | |

**Table 7:** CDR3 sequence analysis in TIL

| TIL ID | Cell subset | Vβ | Vβ | (-D-) | Jβ | Jβ | Flow AB |
|--------|-------------|-----|-----|-------|-----|-----|---------|
| Panc 1 | **CD8** | TRBV9 | CAS | SVQGVSQ | ETQYF | TRBJ2-5 | Vβ1 |
| Panc 5 | **CD4** | TRBV12-3/4 | CAS | SRRGSG | DTQYF | TRBJ2-3 | Vβ8 |
| Panc 7 | **CD4** | TRBV5.1 | CAS | STDSN | TEAFF | TRBJ1-1 | Vβ5.1 |
| | **CD8** | TRBV28 | CAS | SFQTAHT | DTQYF | TRBJ2-3 | Vβ3 |
| Panc 8 | **CD8** | TRBV14 | CAS | SLRDS | DEAFF | TRBJ1-1 | Vβ16 |
| Panc 9 | **CD8** | TRV6-2/3 | CAS | SLQGRV | DEQFF | TRBJ2-1 | NA |
| Pane 16 | CD4 | TRBV20-1 | CSA | RDGTLN | TEAFF | TRBJ1-1 | Vβ2 |
| | | TRBV20-1 | CSA | RPLRDRVA | HGYTF | TRBJ1-2 | Vβ2 |
| | CD8 | TRBV19 | CAS | SILAFRAG | ETQYF | TRBJ2-5 | Vβ17 |
| | | TRBV7-6 | CAS | SQGPN | YEQYF | TRBJ2-7 | NA |
| Pane 17 | CD8 | TRBV7-2 | CAS | SFGLAGA | NEQFF | TRBJ2-1 | NA |
| | | TRBV7-2 | CAS | SSRLAGTY | NEQFF | TRBJ2-1 | NA |

**Example 7 - Recognition of known TAAs by the individual T-cell products from pancreas cancer patients**

TIL were exposed to commonly shared antigens for three days, in the presence of the blocking MHC class I (W6/32) or HLA-DR (L243) mAb, followed by IFNγ production analysis in the supernatant. Strong reactivity to Mesothelin that could be blocked with mAbs. Note that Panc 10 is predominantly a CD4+ TIL that could be blocked with the anti-HLA-DR mAb and not with the MHC class I -directed reagent. We have also included an isotype control (anti-C1q, 242G3 mAb) that showed similar results as the antigen stimulation without the blocking mAb. Results are means of triplicates.

Increased reactivity upon L243 (e.g. Panc16) may reflect T-cell stimulation of crosslinking HLA-DR molecules on activated T-cells. Only positive results are reported.

**Table 8: TAA IFNγ production (pg/mL) in TIL in response to commonly shared TAAs**

| | NY-ESO1 | NY-ESO1 +W6/32 | NY-ESO1 +L243 | Survivin | Survivin +W6/3 2 | Survivin +L243 | Mesothelin | Mesothelin +W6/32 | Mesothelin +L243 |
|---|---|---|---|---|---|---|---|---|---|
| **Panc 1** | 6,97 | 0 | 0 | 0 | 0 | 0 | 262,23 | 15,26 | 0 |
| **Panc 2** | 24,20 | 0 | 0 | 0 | 0 | 0 | 291,27 | 13,50 | 0 |
| **Panc 10** | 4,13 | 0 | 0 | 66,06 | 0 | 0 | 78,70 | 72,88 | 0 |
| **Panc 11** | 0 | 0 | 0 | 0 | 0 | 0 | 131,16 | 0 | 0 |
| **Panc 14** | 10,47 | 0 | 0 | 0 | 0 | 0 | 275,15 | nd | 249,95 |
| **Panc 16** | 173,19 | 0 | 0 | 8,35 | 0 | 0 | 19,64 | 0 | 60,84 |

**REFERENCES**

1. Rosenberg SA, Restifo NP, Yang JC, Morgan RA, Dudley ME. Adoptive cell transfer: a clinical path to effective cancer immunotherapy. Nature reviews Cancer. 2008;8(4):299-308.

2. Rosenberg SA, Packard BS, Aebersold PM, Solomon D, Topalian SL, Toy ST, et al. Use of tumor-infiltrating lymphocytes and interleukin-2 in the immunotherapy of patients with metastatic melanoma. A preliminary report. The New England journal of medicine. 1988;319(25):1676-80.

3. Schwartzentruber DJ, Hom SS, Dadmarz R, White DE, Yannelli JR, SteinbergSM, et al. In vitro predictors of therapeutic response in melanoma patients receiving tumor- infiltrating lymphocytes and interleukin-2. Journal of clinical oncology : official journal of the American Society of Clinical Oncology. 1994;12(7):1475-83.

4. PittetMJ, Speiser DE, Valmori D, Cerottini JC, Romero P. Cutting edge: cytolytic effector function in human circulating CD8+ T cells closely correlates with CD56 surface expression. Journal of immunology. 2000;164(3):1148-52.

5. ShenX, Zhou J, Hathcock KS, Robbins P, Powell DJ, Jr., Rosenberg SA, et al.Persistence of tumor infiltrating lymphocytes in adoptive immunotherapy correlates with telomere length. Journal of immunotherapy. 2007;30(1):123-9.

6. Zhou J, Shen X, Huang J, Hodes RJ, Rosenberg SA, Robbins PF. Telomere length of transferred lymphocytes correlates with in vivo persistence and tumor regression in melanoma patients receiving cell transfer therapy. Journal of immunology. 2005;175(10):7046-52.

7. Green M Sambrook J, Molecular Cloning: A Laboratory Manual, Fourth Edition. Cold Spring Harbor Press. 2012.

8. Wingfield, PT. Production of Recombinant Proteins. Current Protocols in Protein Science. 2010.

9. Robbins PF, Morgan RA, Feldman SA, Yang JC, Sherry RM, Dudley ME, Wunderlich JR, Nahvi AV, Helman LJ, Mackall CL, Kammula US, Hughes MS, Restifo NP, Raffeld M, Lee CC, Levy CL, Li YF, El-Gamil M, Schwarz SL, Laurencot C, Rosenberg SA. Tumor regression in patients with metastatic synovial cell sarcoma and melanoma using genetically engineered lymphocytes reactive with NY-ESO-1. Journal of Clinical Oncology. 2011; 29(7):917-924.

10. Kershaw M, Westwood JA,. Darcy PK. Gene-engineered T cells for cancer therapy. Nature Reviews Cancer, 2013;13:525-541.

11. Johnson LA, Heemskerk B, Powell DJ Jr, Cohen CJ, Morgan RA, Dudley ME, Robbins PF, Rosenberg SA. Gene transfer of tumor-reactive TCR confers both high avidity and tumor reactivity to nonreactive peripheral blood mononuclear cells and tumor-infiltrating lymphocytes. Journal of Immunology. 2006; 177(9):6548-6559.

12. Morgan RA, Dudley ME, Wunderlich JR, Hughes MS, Yang JC, Sherry RM, Royal RE, Topalian SL, Kammula US, Restifo NP, Zheng Z, Nahvi A, de Vries CR, Rogers-Freezer LJ, Mavroukakis SA, Rosenberg SA. Cancer regression in patients after transfer of genetically engineered lymphocytes. Science. 2006;314(5796):126-129.

**Claims**

1. A protein comprising a Vbeta sequence with an amino acid sequence with a sequence identity of at 90 %, to a sequence selected from SEQ ID NO: 1 to 27.

2. The protein according to claim 1, wherein the sequence identity is at least 95 %, more preferably at least 98 %, in particular 100 %.

3. A protein according to claim 1 or 2, wherein the protein is a recombinant protein.

4. The protein according to any of claims 1 to 3, wherein the protein is a TCR.

5. A T-cell comprising a TCR according to claim 4.

6. The T-cell according to claim 5, for use in the treatment of cancer.

7. A method of selecting a T-cell product for use in active immunotherapy in a patient, wherein the method comprises the following steps:

a) providing at one or more T-cell products;
b) identifying the presence of a TCR comprising a sequence with a homology of at least 95 % to the sequence selected from SEQ ID NO: 1 to 27in the one or more T-cell products; and
c) selecting the T-cell product comprising a TCR identified in step b) for use in therapy.

8. The method according to claim 7, wherein the T-cell product is a T-cell population

9. The method according to claim 7 or 8, further comprising determining the clonality of the Vβ family to which the TCR belongs and selecting the T-cell product in which the TCR is present and the Vβ family to which the TCR belongs is monoclonal.

10. The method according to claim 7 to 9, further comprising determining the percentage of the TCR and/or the Vβ family to which the TCR belongs in the T-cell product and selecting the T-cell product in which the percentage of the TCR is the highest.

11. The method according to any of claims 7 to 10, wherein the patient suffers from cancer.

12. The method according to any of claims 7 to 11, wherein the cancer is selected from glioblastoma and pancreas cancer.

13. The method according to any of claims 7 to 12, wherein providing the one or more T-cell products comprises:

- providing a body sample containing T-cells from the patient;
- culturing cells of the sample *in-vitro* in the presence of at least one cytokine, preferably in the presence of interleukin 2 (IL-2), interleukin 15 (IL-15) and interleukin 21 (IL-21), and optionally an expansion antigen to expand the T-cells.

Fig. 1

Fig. 2

Fig. 3

Fig. 4

Europäisches
Patentamt

European
Patent Office

Office européen
des brevets

# EUROPEAN SEARCH REPORT

**Application Number**

EP 16 15 4713

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2012/038055 A1 (UNICELL GMBH [DE]; UNIVERSITAETSMEDIZIN DER JOHANNES GUTENBERG UNI MAI) 29 March 2012 (2012-03-29) | 1-6 | INV. C07K14/725 A61K38/20 |
| Y | * abstract; claim 14; example 1 * & DATABASE Sequence ID [Online] Omokoko T et al: "Human NY-ESO-1 specific T cell receptor (TCR) beta chain, SEQ ID 143", Database accession no. AZU40714 ----- | 7-13 | |
| X | WO 2005/056595 A2 (IMP COLLEGE INNOVATIONS LTD [GB]; STAUSS HANS JOSEF [GB]; GAO LIQUAN []) 23 June 2005 (2005-06-23) | 1-6 | |
| Y | * abstract; claims 1,22; figures 8,9 * & DATABASE GSP [Online] Stauss HJ et al.: "Wilm's tumor antigen-1 (WT1) peptide TCR beta chain.", Database accession no. AEA49987 ----- | 7-13 | |
| X | WO 2014/083173 A1 (MAX DELBRÜCK CT FÜR MOLEKULARE MEDIZIN MDC BERLIN BUCH [DE]) 5 June 2014 (2014-06-05) | 1-6 | TECHNICAL FIELDS SEARCHED (IPC) C07K C12N A61K |
| Y | * pages 9-10 * & DATABASE GSP [Online] Blankenstein T et al.: "RAC1 specific T cell receptor beta chain variable domain, SEQ ID 48", Database accession no. BBH06876 ----- | 7-13 | |
| Y | WO 2015/189356 A1 (POLYBIOCEPT AB [SE]) 17 December 2015 (2015-12-17) * claims 1-7,35-36; examples 23, 24 * ----- | 1-13 | |

-/--

~~The present search report has been drawn up for all claims~~

| Place of search | Date of completion of the search | Examiner |
|---|---|---|
| Munich | 22 April 2016 | Novak-Giese, Sabine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 16 15 4713

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SHI LI ET AL: "Enhancement of the TCR[zeta] expression, polyclonal expansion, and activation of t cells from patients with acute myeloid leukemia after IL-2, IL-7, and IL-12 induction.", DNA AND CELL BIOLOGY JUL 2015, vol. 34, no. 7, July 2015 (2015-07), pages 481-488, XP055266345, ISSN: 1557-7430 * page 486, paragraph 1 * | 1-13 | |
| Y | AHMED RAIJA K ET AL: "TCR+CD4-CD8- T cells in antigen-specific MHC class I-restricted T-cell responses after allogeneic hematopoietic stem cell transplantation.", JOURNAL OF IMMUNOTHERAPY (HAGERSTOWN, MD. : 1997) OCT 2014, vol. 37, no. 8, October 2014 (2014-10), pages 416-425, XP009189604, ISSN: 1537-4513 * page 418, paragraph 3 * | 1-13 | |

TECHNICAL FIELDS
SEARCHED (IPC)

~~The present search report has been drawn up for all claims~~

| Place of search | Date of completion of the search | Examiner |
|---|---|---|
| Munich | 22 April 2016 | Novak-Giese, Sabine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-13(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 16 15 4713

The Search Division considers that the present European patent application does not comply with the requirements of unity of invention and relates to several inventions or groups of inventions, namely:

1. claims: 1-13(partially)

   A protein comprising a Vbeta sequence with an amino acid sequence with an identity of at least 90% to SEQ ID NO:1. Also encompassed are T cells comprising a TCR with said sequence and such T cells for use in the treatment of cancer. Further encompassed are methods of selecting 'T cell products' for use in immunotherapy by identifying the presence of such proteins in the TCR and using said T cell products in therapy.

   ---

2-26. claims: 1-13(partially)

   A protein comprising a Vbeta sequence with an amino acid sequence with an identity of at least 90% to SEQ ID NO:2-26. Also encompassed are T cells comprising a TCR with said sequence and such T cells for use in the treatment of cancer. Further encompassed are methods of selecting 'T cell products' for use in immunotherapy by identifying the presence of such proteins in the TCR and using said T cell products in therapy.

   ---

## ANNEX TO THE EUROPEAN SEARCH REPORT
## ON EUROPEAN PATENT APPLICATION NO.

EP 16 15 4713

This annex lists the patent family members relating to the patent documents cited in the above-mentioned European search report.
The members are as contained in the European Patent Office EDP file on
The European Patent Office is in no way liable for these particulars which are merely given for the purpose of information.

22-04-2016

| Patent document cited in search report | | | Publication date | Patent family member(s) | | | Publication date |
|---|---|---|---|---|---|---|---|
| WO 2012038055 | A1 | | 29-03-2012 | AU | 2011304728 | A1 | 14-03-2013 |
| | | | | CA | 2812153 | A1 | 29-03-2012 |
| | | | | CN | 103249430 | A | 14-08-2013 |
| | | | | CN | 105255834 | A | 20-01-2016 |
| | | | | EP | 2618835 | A1 | 31-07-2013 |
| | | | | JP | 2013541332 | A | 14-11-2013 |
| | | | | JP | 2016136939 | A | 04-08-2016 |
| | | | | US | 2013273647 | A1 | 17-10-2013 |
| | | | | WO | 2012038055 | A1 | 29-03-2012 |
| WO 2005056595 | A2 | | 23-06-2005 | EP | 1692172 | A2 | 23-08-2006 |
| | | | | EP | 2913340 | A1 | 02-09-2015 |
| | | | | ES | 2534224 | T3 | 20-04-2015 |
| | | | | HK | 1214280 | A1 | 22-07-2016 |
| | | | | US | 2007191496 | A1 | 16-08-2007 |
| | | | | US | 2011274675 | A1 | 10-11-2011 |
| | | | | US | 2015147302 | A1 | 28-05-2015 |
| | | | | WO | 2005056595 | A2 | 23-06-2005 |
| WO 2014083173 | A1 | | 05-06-2014 | AU | 2013351062 | A1 | 04-06-2015 |
| | | | | CA | 2891967 | A1 | 05-06-2014 |
| | | | | CN | 104853765 | A | 19-08-2015 |
| | | | | EP | 2925349 | A1 | 07-10-2015 |
| | | | | GB | 2508414 | A | 04-06-2014 |
| | | | | HK | 1214942 | A1 | 12-08-2016 |
| | | | | JP | 2015536665 | A | 24-12-2015 |
| | | | | US | 2015307585 | A1 | 29-10-2015 |
| | | | | WO | 2014083173 | A1 | 05-06-2014 |
| WO 2015189356 | A1 | | 17-12-2015 | WO | 2015189356 | A1 | 17-12-2015 |
| | | | | WO | 2015189357 | A1 | 17-12-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015189357 A1

**Non-patent literature cited in the description**

- **NEEDLEMAN ; WUNSCH.** *J. Mol. Biol.,* 1970, vol. 48, 443-453
- **RICE.** EMBOSS: The European Molecular Biology Open Software Suite. *Trends Genet,* 2000, vol. 16, 276-277
- **ROSENBERG SA ; RESTIFO NP ; YANG JC ; MORGAN RA ; DUDLEY ME.** Adoptive cell transfer: a clinical path to effective cancer immunotherapy. *Nature reviews Cancer,* 2008, vol. 8 (4), 299-308
- **ROSENBERG SA ; PACKARD BS ; AEBERSOLD PM ; SOLOMON D ; TOPALIAN SL ; TOY ST et al.** Use of tumor-infiltrating lymphocytes and interleukin-2 in the immunotherapy of patients with metastatic melanoma. A preliminary report. *The New England journal of medicine,* 1988, vol. 319 (25), 1676-80
- **SCHWARTZENTRUBER DJ ; HOM SS ; DADMARZ R ; WHITE DE ; YANNELLI JR ; STEINBERGSM et al.** In vitro predictors of therapeutic response in melanoma patients receiving tumor- infiltrating lymphocytes and interleukin-2. *Journal of clinical oncology : official journal of the American Society of Clinical Oncology,* 1994, vol. 12 (7), 1475-83
- **PITTETMJ ; SPEISER DE ; VALMORI D ; CEROTTINI JC ; ROMERO P.** Cutting edge: cytolytic effector function in human circulating CD8+ T cells closely correlates with CD56 surface expression. *Journal of immunology,* 2000, vol. 164 (3), 1148-52
- **SHENX, ZHOU J ; HATHCOCK KS ; ROBBINS P ; POWELL DJ, JR. ; ROSENBERG SA et al.** Persistence of tumor infiltrating lymphocytes in adoptive immunotherapy correlates with telomere length. *Journal of immunotherapy,* 2007, vol. 30 (1), 123-9

- **ZHOU J ; SHEN X ; HUANG J ; HODES RJ ; ROSENBERG SA ; ROBBINS PF.** Telomere length of transferred lymphocytes correlates with in vivo persistence and tumor regression in melanoma patients receiving cell transfer therapy. *Journal of immunology,* 2005, vol. 175 (10), 7046-52
- **GREEN M ; SAMBROOK J.** Molecular Cloning: A Laboratory Manual. Cold Spring Harbor Press, 2012
- **WINGFIELD, PT.** Production of Recombinant Proteins. *Current Protocols in Protein Science,* 2010
- **ROBBINS PF ; MORGAN RA ; FELDMAN SA ; YANG JC ; SHERRY RM ; DUDLEY ME ; WUNDERLICH JR ; NAHVI AV ; HELMAN LJ ; MACKALL CL.** Tumor regression in patients with metastatic synovial cell sarcoma and melanoma using genetically engineered lymphocytes reactive with NY-ESO-1. *Journal of Clinical Oncology,* 2011, vol. 29 (7), 917-924
- **KERSHAW M ; WESTWOOD JA ; DARCY PK.** Gene-engineered T cells for cancer therapy. *Nature Reviews Cancer,* 2013, vol. 13, 525-541
- **JOHNSON LA ; HEEMSKERK B ; POWELL DJ JR ; COHEN CJ ; MORGAN RA ; DUDLEY ME ; ROBBINS PF ; ROSENBERG SA.** Gene transfer of tumor-reactive TCR confers both high avidity and tumor reactivity to nonreactive peripheral blood mononuclear cells and tumor-infiltrating lymphocytes. *Journal of Immunology,* 2006, vol. 177 (9), 6548-6559
- **MORGAN RA ; DUDLEY ME ; WUNDERLICH JR ; HUGHES MS ; YANG JC ; SHERRY RM ; ROYAL RE ; TOPALIAN SL ; KAMMULA US ; RESTIFO NP.** Cancer regression in patients after transfer of genetically engineered lymphocytes. *Science,* 2006, vol. 314 (5796), 126-129